# EUROPEAN PATENT SPECIFICATION

(11) **EP 4 602 608 B1**
(45) Date of publication and mention of the grant of the patent: **02.12.2026**
(21) Application number: 23805779.8
(22) Date of filing: 10.10.2023
(51) Int. Cl.: G16B 20/20, G16B 30/00, G16B 30/10

(54) **DETECTING AND CORRECTING METHYLATION VALUES FROM METHYLATION SEQUENCING ASSAYS**
NACHWEIS UND KORREKTUR VON METHYLIERUNGSWERTEN AUS METHYLIERUNGSSEQUENZIERUNGSASSAYS
DÉTECTION ET CORRECTION DE VALEURS DE MÉTHYLATION À PARTIR DE DOSAGES DE SÉQUENÇAGE DE MÉTHYLATION

(30) Priority: 11.10.2022 US 202263379095 P
(43) Date of publication of application: 20.08.2025
(73) Proprietor: Illumina, Inc., San Diego, CA 92122 (US)
(72) Inventor: WANG, Qi, San Diego, California 92122 (US); ROHRBACK, Suzanne, San Diego, California 92122 (US); SHULTZABERGER, Sarah, San Diego, California 92122 (US); KARADEEMA, Rebekah, San Diego, California 92122 (US); MING, Leslie Beh Yee, Singapore 757716 (SG); BAYE, James, Cambridge CB21 6DF (GB); BROWN, Colin, San Diego, California 92122 (US)
(74) Representative: Robinson, David Edward Ashdown
(86) International application number: PCT/US2023/076472
(87) International publication number: WO 2024/081649

(56) References cited:
- WO-A1-2021/032060
- WO-A1-2021/258026
- US-A1- 2017 362 638
- US-A1- 2021 230 684

## Description

### BACKGROUND

In recent years, biotechnology firms and research institutions have improved hardware and software for both detecting methylation of cytosine bases at particular genomic regions (e.g., regions encoding or promoting genes) and detecting methylation of larger nucleotide fragments or whole genomes of a sample. For instance, some existing sequencing systems can use sequencing devices and corresponding sequencing-data-analysis software to identify when a methyl or hydroxymethyl group has been added to a cytosine base of a sample's deoxyribonucleic acid (DNA)-where the methylated cytosine base is often part of a cytosine-guanine-dinucleotide pair in a 5'-C-phosphate-G-3' (CpG) configuration in mammals. For example, existing sequencing systems can detect methylated cytosines by (i) enzymatically converting methylated or unmethylated cytosine bases at CpG or other sites from a sample nucleotide fragment into uracil bases (e.g., dihydrouracil); (ii) determining base calls of nucleotide reads for the sample using a sequencing device, where the sequencing device detects the uracil bases as thymine bases during polymerase chain reaction (PCR) amplification; and (iii) comparing the base calls from the nucleotide reads to a reference genome or non-enzymatically converted nucleotide reads from the sample. Based on the comparison of nucleotide reads from the sample to a reference genome or the non-enzymatically converted nucleotide reads, existing sequencing systems can identify thymine bases from the nucleotide reads that do not match cytosine bases at CpG or other sites within the reference genome or the non-enzymatically converted nucleotide reads and thereby detect methylated cytosine bases in a sample nucleotide fragment.

To convert cytosine to uracil, in some cases, existing methylation assays use bisulfite as an enzyme, whereas other methylation assays use a non-bisulfite enzyme. For instance, Tet-assisted pyridine borane sequencing (TAPS) uses a ten-eleven translocation (TET) enzyme for a methylation assay, as described by Yibin Liu et al., "Bisulfite-free Direct Detection of 5-Methylcystosine and 5-Hydroxymethylcystosine at Base Resolution," 36 Nature Biotechnology 424-29 (2019). In some assays that rely on a TET enzyme, a methylation sequencing assay applies converts 5-Methylcystosine (5mC) and 5-Hydroxymethylcystosine (5hmC) into oxidized products using a TET enzyme and then uses an Apolipoprotein B mRNA Editing Enzyme, Catalytic Polypeptide (APOBEC) 3A or other APOBEC protein to deaminate unmodified cytosines by converting them to uracil bases. While bisulfite can be more reliable as a conversion enzyme than some other enzymes, bisulfite can also adversely affect other components of a nucleotide fragment for sequencing.

By performing methylation sequencing assays using various enzymes or approaches, existing sequencing systems can detect cytosine methylation for various diagnostic or therapeutic purposes. For example, some existing sequencing systems can perform a methylation assay to determine methylation levels of CpG islands that span gene promoters or exons and thereby use CpG methylation detection as an early biomarker of cancer. Existing methylation assays can similarly be used to determine methylation levels of certain genomic regions for genes relevant to other diseases, such as certain autoimmune, neurological, and psychiatric disorders. In addition to using methylation assays to detect disorders, in some cases, existing sequencing systems perform a methylation assay to determine methylation levels of promoter regions and determine how such methylation affects regulation and expression of genes corresponding to promoter regions.

Despite recent improvements to methylation sequencing assays, existing sequencing systems often generate inaccurate methylation measurements for cytosine bases. For example, existing methylation sequencing assays often generate beta values or M values indicating a level of cytosine methylation at particular genomic coordinates or regions. But such beta values or M values can misrepresent actual levels of cytosine methylation in a DNA fragment. As described further below, existing methylation assays can determine beta values or M values for cytosine bases at specific genomic sites with accuracies that vary wildly.

Because methylation levels can be a biomarker for cancer or other diseases, in some cases, existing sequencing systems determine and leverage beta values or M values indicating levels of cytosine methylation as biological signals for certain diseases. For example, some sequencing systems determine beta values in a differentially methylated region (DMR) for a sample and interpret the beta values across the DMR as bio signals for cancer. Despite the utility and importance of methylation levels in such regions as biomarkers, existing methylation sequencing assays must but subject to correction methods to adjust for inaccurate methylation-level measurements. Nevertheless, correction methods, such as comprehensive high-throughput arrays for relative methylation (CHARM), recover approximately 70% of biological signals when using Enzymatic Methyl (EM)-Seq for ground-truth data.

Because existing sequencing systems execute methylation assays that are inaccurate or otherwise unreliable, existing systems may re-run methylation assays on multiple copies of DNA fragments from a sample or run different types of methylation assays to determine more reliable beta values or M values for consensus. But such re-execution of methylation assays or use of different methylation-assay types can consume valuable computing resources on both specialized sequencing devices and computing devices executing sequencing-data-analysis software-thereby performing redundant analyses and performing time-intensive-computer processing on such computing devices, where sequencing runs alone can consume between approximately four to fifty-five hours on specialized sequencing devices. Despite the importance and extreme variability of such methylation assays, the technical cause of some of existing methylation assays' variation and inaccuracies have been unclear and puzzling prior to this disclosure.

To adjust and correct inaccurate methylation-level values, some existing systems apply filters to remove reads with problematic conversion results. For example, a publicly available software application called Bismark Bisulfite Mapper includes a filter that removes, from a sample's data, reads comprising cytosines that bisulfite failed or incompletely converted into uracil. But Bismark works exclusively on bisulfite-based methylation sequencing assays and expends considerable computer processing to analyze and identify reads comprising incompletely converted cytosines. Nor is there publicly available data indicating the degree to which the read filtering improves beta or M values detecting methylation.

### SUMMARY

This disclosure describes one or more embodiments of systems, methods, and non-transitory computer readable storage media that solve one or more of the problems described above or provide other advantages over the art. In particular, the disclosed system uses a computationally efficient model to determine a corrected methylation-level value for a specific sample nucleotide sequence. For instance, the disclosed systems determine a false positive rate and a false negative rate at which a given methylation sequencing assay converts cytosine bases. Based on the determined false positive rate and false negative rate, the disclosed systems determine a corrected methylation-level value that corrects for a bias of the given methylation sequencing assay.

To illustrate but one embodiment, in some cases, the disclosed system identifies a methylation-level value determined by a given methylation sequencing assay for a target cytosine base within a sample nucleotide sequence. The disclosed system determines a false positive rate and false negative rate at which the given methylation sequencing assay converts cytosine bases within nucleotide sequences into uracil bases. Based on the false positive rate and the false negative rate, the disclosed system predicts a first corrected number of nucleotide reads supporting methylated cytosine sites within the sample nucleotide sequence and a second corrected number of nucleotide reads supporting unmethylated cytosine sites within the sample nucleotide sequence. Based on first number and second corrected number of supporting nucleotide reads, the disclosed fragments from a sample or run different types of methylation assays to determine more reliable beta values or M values for consensus. But such re-execution of methylation assays or use of different methylation-assay types can consume valuable computing resources on both specialized sequencing devices and computing devices executing sequencing-data-analysis software-thereby performing redundant analyses and performing time-intensive-computer processing on such computing devices, where sequencing runs alone can consume between approximately four to fifty-five hours on specialized sequencing devices. Despite the importance and extreme variability of such methylation assays, the technical cause of some of existing methylation assays' variation and inaccuracies have been unclear and puzzling prior to this disclosure.

To adjust and correct inaccurate methylation-level values, some existing systems apply filters to remove reads with problematic conversion results. For example, a publicly available software application called Bismark Bisulfite Mapper includes a filter that removes, from a sample's data, reads comprising cytosines that bisulfite failed or incompletely converted into uracil. But Bismark works exclusively on bisulfite-based methylation sequencing assays and expends considerable computer processing to analyze and identify reads comprising incompletely converted cytosines. Nor is there publicly available data indicating the degree to which the read filtering improves beta or M values detecting methylation.

### SUMMARY

This disclosure describes one or more embodiments of systems, methods, and non-transitory computer readable storage media that solve one or more of the problems described above or provide other advantages over the art. In particular, the disclosed system uses a computationally efficient model to determine a corrected methylation-level value for a specific sample nucleotide sequence. For instance, the disclosed systems determine a false positive rate and a false negative rate at which a given methylation sequencing assay converts cytosine bases. Based on the determined false positive rate and false negative rate, the disclosed systems determine a corrected methylation-level value that corrects for a bias of the given methylation sequencing assay.

To illustrate but one embodiment, in some cases, the disclosed system identifies a methylation-level value determined by a given methylation sequencing assay for a target cytosine base within a sample nucleotide sequence. The disclosed system determines a false positive rate at which the given methylation sequencing assay converts cytosine bases within nucleotide sequences by comparing (i) a number of converted unmethylated cytosine bases within an unmethylated artificial oligonucleotide subject to the methylation sequencing assay to (ii) a total number of unmethylated cytosine bases within the unmethylated artificial oligonucleotide and determines a false negative rate at which the methylation sequencing assay converts cytosine bases within nucleotide sequences by comparing (i) a number of unconverted methylated cytosine bases within a methylated artificial oligonucleotide subject to the methylation sequencing assay to (ii) a total number of methylated cytosine bases within the methylated artificial oligonucleotide. Based on a first quotient comparing the false positive rate to the false negative rate, the disclosed system predicts a first corrected number of nucleotide reads supporting methylated cytosine sites within the sample nucleotide sequence and based on a second quotient comparing the false negative rate to the false positive rate, the disclosed system predicts a second corrected number of nucleotide reads supporting unmethylated cytosine sites within the sample nucleotide sequence. The disclosed system generates a corrected methylation-level value that corrects for a bias reflected in the methylation-level value for the target cytosine base within the sample nucleotide sequence by determining a third quotient comparing (i) the first corrected number of nucleotide reads to (ii) the first corrected number of nucleotide reads and the second corrected number of nucleotide reads. By using the corrected methylation-level value, the disclosed system can likewise recover biological signals for cancer, Alzheimer's, and other methylation-dependent diseases.

### BRIEF DESCRIPTION OF THE DRAWINGS

The detailed description refers to the drawings briefly described below.

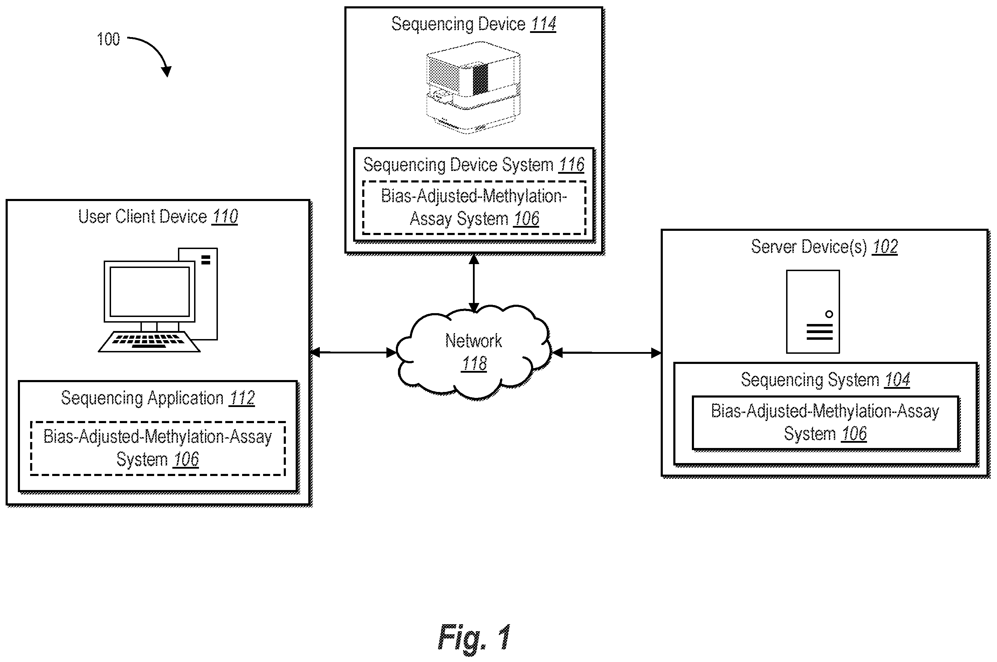

FIG. 1 illustrates a computing-system environment in which a bias-adjusted-methylation-assay system can operate in accordance with one or more embodiments of the present disclosure.

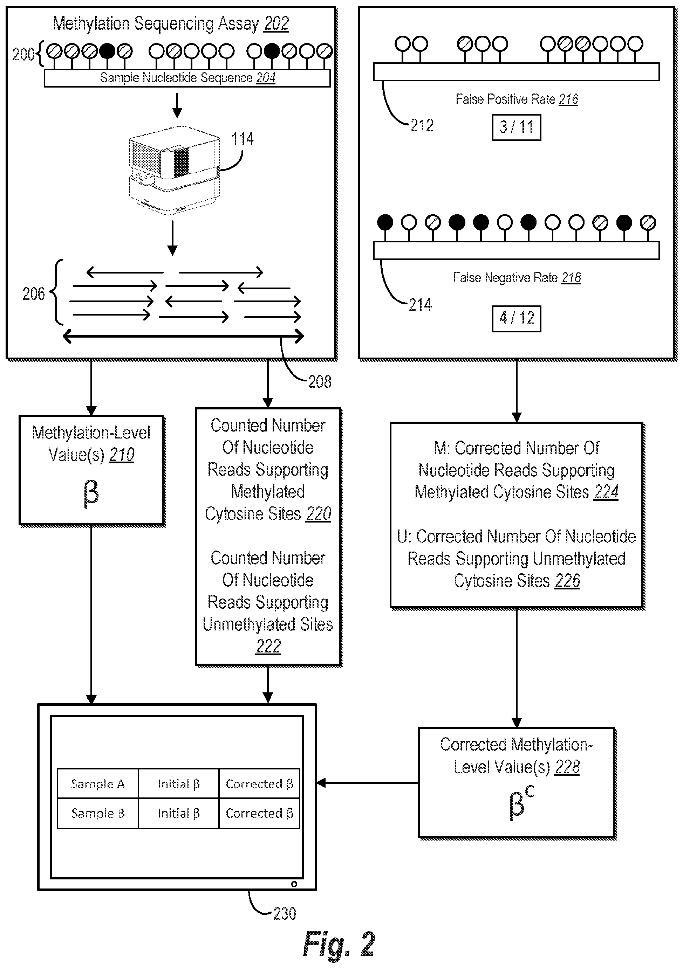

FIG. 2 illustrates a schematic diagram of the bias-adjusted-methylation-assay system identifying, for a given methylation sequencing assay, an initial methylation-level value for a target cytosine base within a sample nucleotide sequence and determining a corrected methylation-level value for the target cytosine base in accordance with one or more embodiments of the present disclosure.

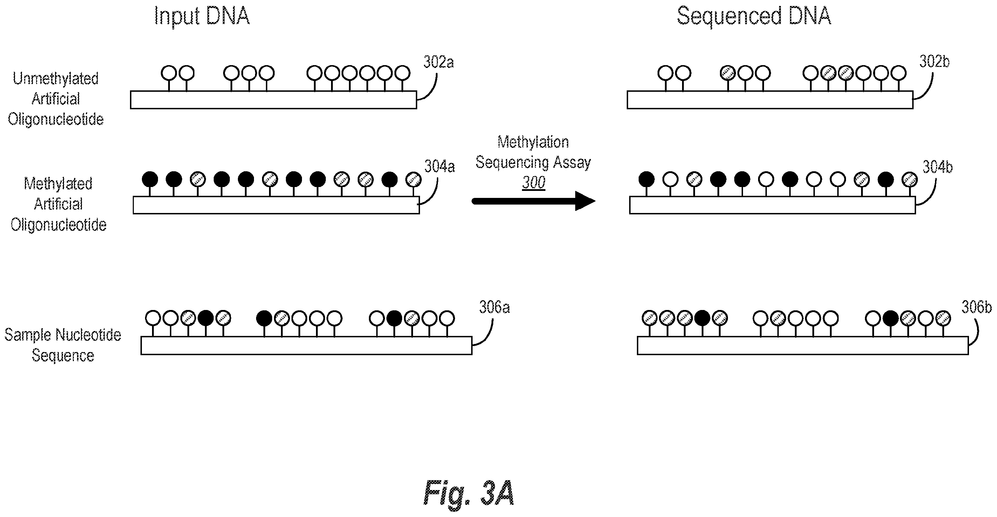

FIGS. 3A-3E illustrate schematic diagrams of the bias-adjusted-methylation-assay system determining corrected methylation-level values specific to both a given methylation sequencing assay and specific to target cytosine bases within sample nucleotide sequences in accordance with one or more embodiments of the present disclosure.

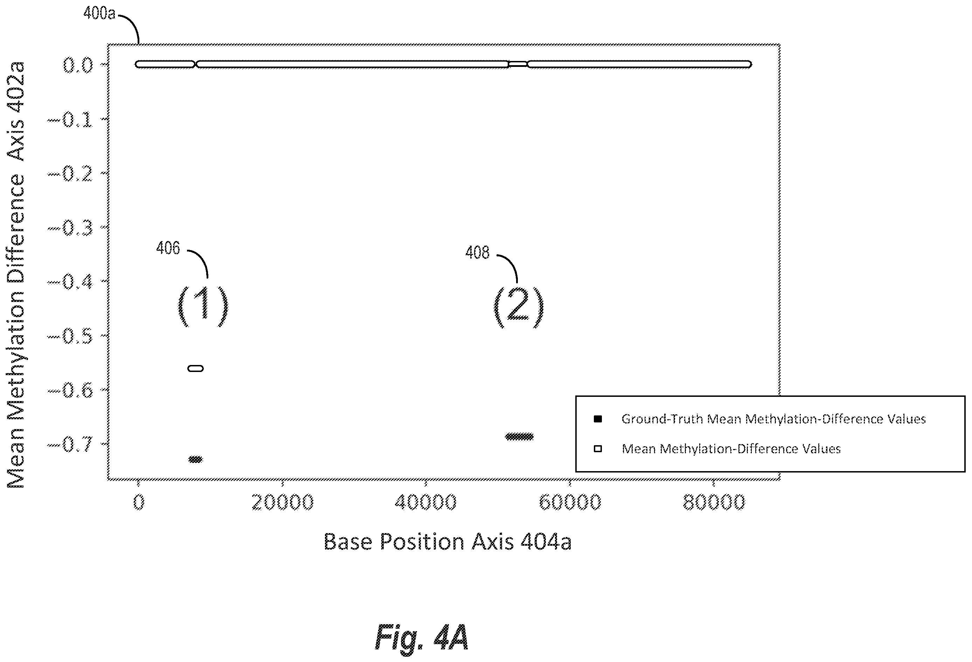

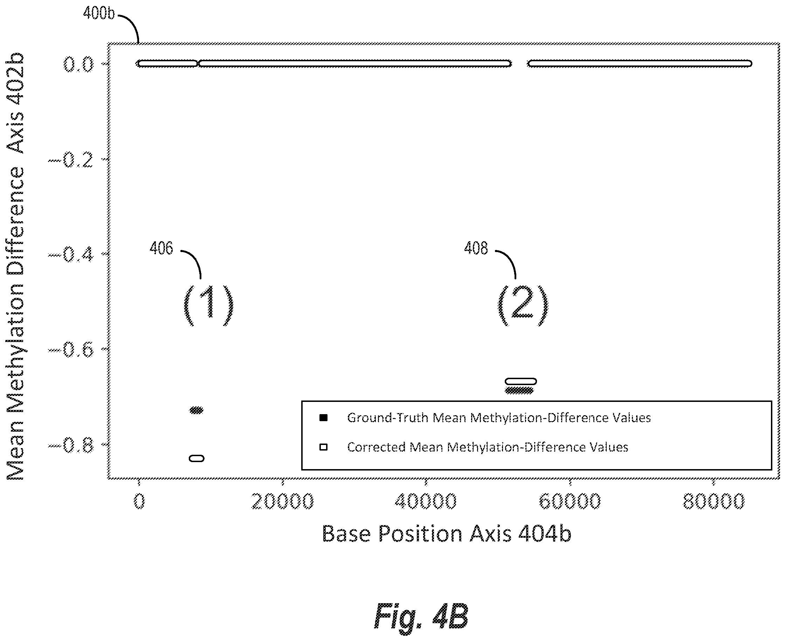

FIGS. 4A and 4B illustrate the bias-adjusted-methylation-assay system modifying methylation-difference values for differentially methylated regions (DMRs) corresponding to target cytosine bases within a sample nucleotide sequence based on corrected methylation-level values in accordance with one or more embodiments of the present disclosure.

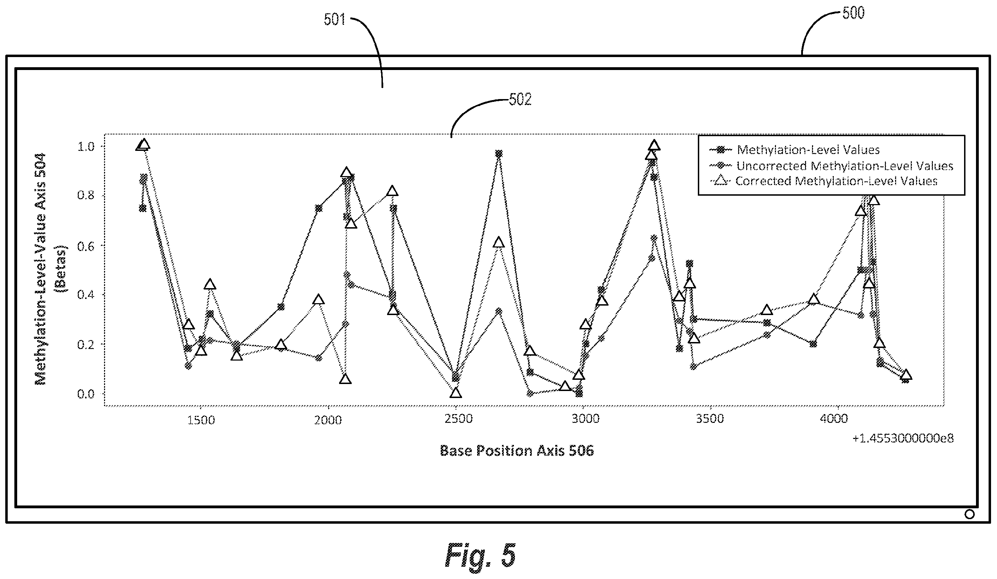

FIG. 5 illustrates a computing device presenting, within a graphical user interface, data representing initial or uncorrected methylation-level values determined by a methylation-sequencing assay and corrected methylation-level values determined by the bias-adjusted-methylation-assay system 106 in accordance with one or more embodiments of the present disclosure.

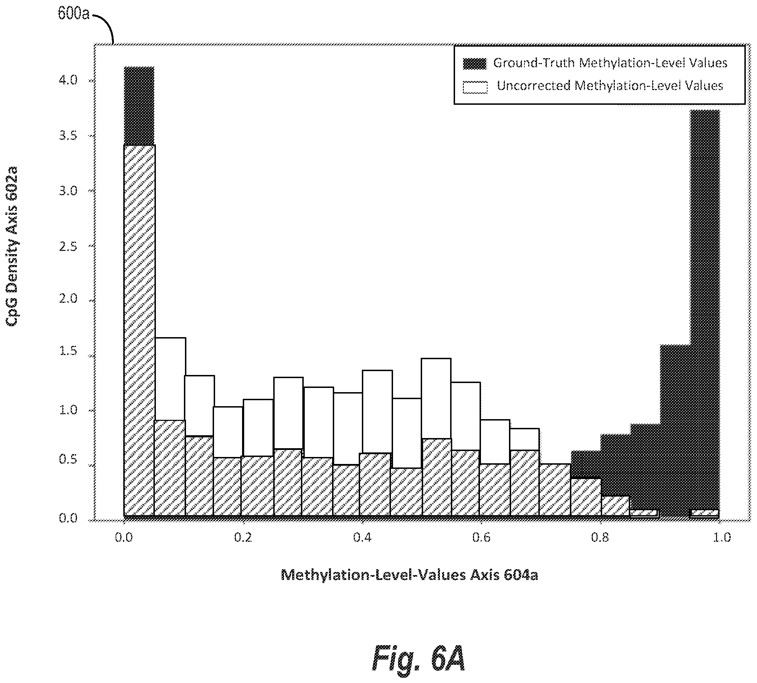

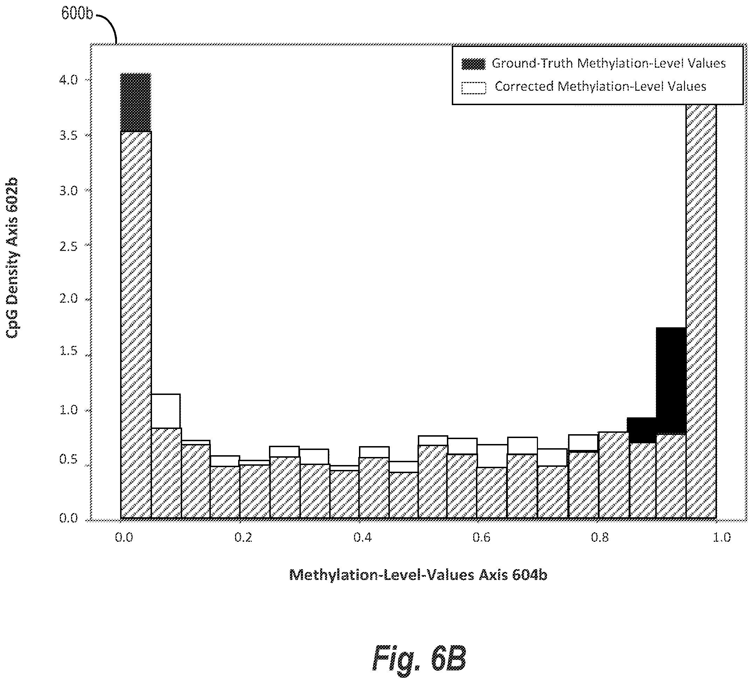

FIGS. 6A and 6B illustrate histograms comparing either uncorrected methylation-level values determined by a given methylation sequencing assay or corrected methylation-level values determined by the bias-adjusted-methylation-assay system to ground-truth methylation-level values across genomic regions of a chromosome in accordance with one or more embodiments of the present disclosure.

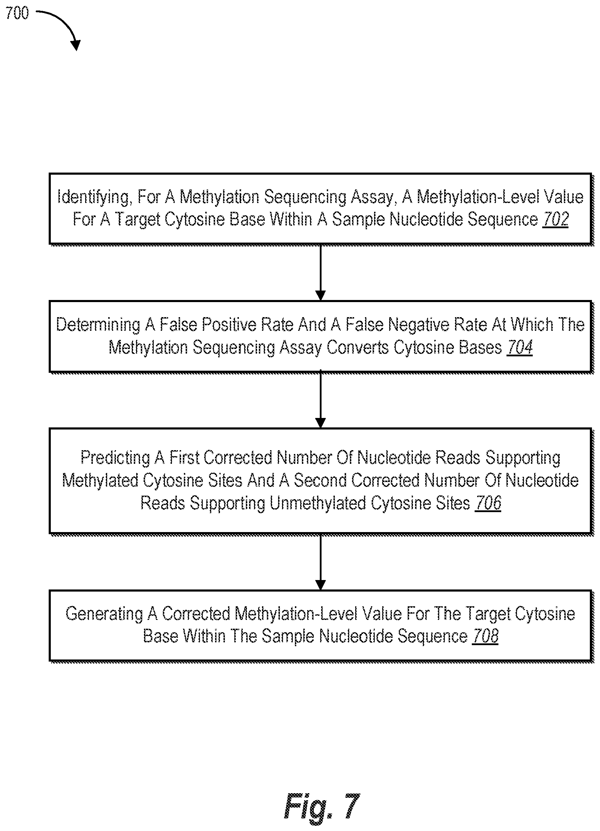

FIG. 7 illustrate a series of acts for utilizing a computational model to determine a corrected methylation-level value for a target cytosine base within a sample nucleotide sequence in accordance with one or more embodiments of the present disclosure.

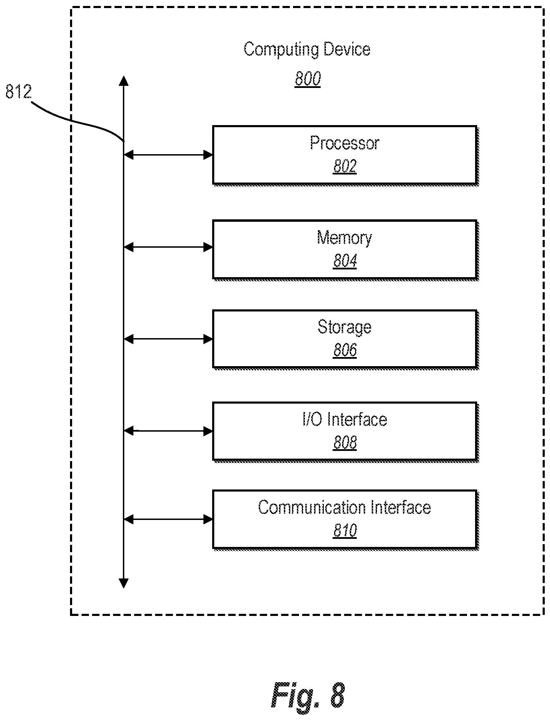

FIG. 8 illustrates a block diagram of an example computing device in accordance with one or more embodiments of the present disclosure.

### DETAILED DESCRIPTION

This disclosure describes one or more embodiments of a bias-adjusted-methylation-assay system that uses a computationally efficient model to determine corrected methylation-level values for specific sample nucleotide sequences analyzed by a given methylation sequencing assay. For instance, the bias-adjusted-methylation-assay system determines a false positive rate and a false negative rate at which a given methylation sequencing assay converts cytosine bases into uracil bases or thymine bases. To determine such rates, the bias-adjusted-methylation-assay system can either identify historical false positive and false negative rates predetermined for the given methylation sequencing assay or determine the false positive and false negative rates by running artificial oligonucleotides with known unmethylated and methylated cytosine bases through the given methylation sequencing assay. Based on the determined false positive and false negative rates, the bias-adjusted-methylation-assay system determines a corrected methylation-level value that corrects for a bias of the given methylation sequencing assay with respect to a target cytosine base in a sample nucleotide sequence. Accordingly, the corrected methylation-level value can be specific to both the given methylation sequencing assay and the sample nucleotide sequence.

To execute the new computational model, in some cases, the bias-adjusted-methylation-assay system identifies a methylation-level value determined by a given methylation sequencing assay for a target cytosine base within a sample nucleotide sequence. The bias-adjusted-methylation-assay system further determines a false positive rate and a false negative rate at which the given methylation sequencing assay converts cytosine bases within nucleotide sequences into uracil bases. Based on the false positive rate and the false negative rate, the bias-adjusted-methylation-assay system predicts (i) a first corrected number of nucleotide reads supporting methylated cytosine sites within the sample nucleotide sequence and (ii) a second corrected number of nucleotide reads supporting unmethylated cytosine sites within the sample nucleotide sequence. Based on the first corrected number and the second corrected number of supporting nucleotide reads, the bias-adjusted-methylation-assay system generates a corrected methylation-level value that corrects for a bias reflected in the methylation-level value for the target cytosine base within the sample nucleotide sequence.

To determine a false negative rate and a false positive rate of a given methylation sequencing assay's cytosine conversion, in some embodiments, the bias-adjusted-methylation-assay system uses artificial oligonucleotides with either known methylated or known unmethylated cytosine sites. To determine the false positive rate, for instance, the bias-adjusted-methylation-assay system runs an unmethylated spike-in oligonucleotide through a methylation sequencing assay to determine a number of converted unmethylated cytosine bases from the unmethylated spike-in oligonucleotide and (ii) compare the number of converted unmethylated cytosine bases to a total number of the unmethylated cytosine bases within the unmethylated spike-in oligonucleotide. Similarly, to determine the false negative rate, the bias-adjusted-methylation-assay system can run a methylated spike-in oligonucleotide through the methylation sequencing assay to determine a number of converted methylated cytosine bases from the methylated spike-in oligonucleotide and (ii) compare the number of converted methylated cytosine bases to a total number of the methylated cytosine bases within the methylated spike-in oligonucleotide.

In addition or in the alternative to being methylation-sequencing-assay specific or sample-nucleotide-sequence specific, in some embodiments, the bias-adjusted-methylation-assay system generates a corrected methylation-level value that is specific to a contextual sequence flanking a target cytosine base. For example, the bias-adjusted-methylation-assay system can (i) estimate the false positive rate or the false negative rate at which the given methylation sequencing assay converts cytosine bases flanked by a contextual sequence and (ii) generate the corrected methylation-level value for the target cytosine base specific to the contextual sequence flanking the target cytosine base. Such a corrected methylation-level value can, therefore, constitute a contextual-sequence-specific corrected methylation-level value.

Beyond being computationally efficient, in some embodiments, the bias-adjusted-methylation-assay system generates a corrected methylation-level value based on a read-coverage trigger-that is, a trigger for generating a corrected methylation-level value only when nucleotide-read coverage for a target cytosine base does not satisfy a threshold coverage. As an example of such coverage-aware correction, the bias-adjusted-methylation-assay system can (i) determine that a number of reads covering the target cytosine base fails to satisfy a coverage threshold and (ii) generate the corrected methylation-level value for the target cytosine base only when such a coverage threshold is not satisfied.

As indicated above, the bias-adjusted-methylation-assay system provides several technical advantages relative to existing sequencing systems, such as by improving the accuracy, computing efficiency, and flexibility of methylation sequencing assays or assay correction models. For instance, in some embodiments, the bias-adjusted-methylation-assay system improves the accuracy of detecting methylation levels of cytosine bases within a sample nucleotide sequence. As suggested above, some existing sequencing systems generate inaccurate methylation-level values (e.g., beta values, M values) that misrepresent actual methylation of particular cytosine bases. As discovered by the inventors of this disclosure, however, a relatively simple and computationally efficient model can correct for a bias reflected by the methylation-level values determined by a given methylation sequencing assay. By determining an estimated false positive rate and an estimated false negative rate at which a given methylation sequencing assay converts cytosine bases-and from such rates predict corrected numbers of nucleotide reads supporting methylated and unmethylated cytosine sites within the sample nucleotide sequence-the bias-adjusted-methylation-assay system can generate a corrected methylation-level value for a specific sample nucleotide sequence that better represents with ground-truth methylation than existing methylation sequencing assays. Indeed, the bias-adjusted-methylation-assay system can generate corrected methylation-level values that correct for failed enzymatic methylation, failed or inaccurate imagining on a sequencing device, or other mechanical or computational errors that hinder existing methylation sequencing assays from accurately detecting methylation levels.

Beyond improved accuracy for methylation-level values, in some embodiments, the bias-adjusted-methylation-assay system recovers biological signals for certain disorders or diseases that would otherwise be missed by existing methylation sequencing assays. For instance, in some cases, the bias-adjusted-methylation-assay system recovers biological signals for cancer, Alzheimer's, and other methylation-dependent diseases. To illustrate, based on a corrected methylation-level value, the bias-adjusted-methylation-assay system can change a methylation-difference value for a differentially methylated region (DMR) corresponding to one or more target cytosine bases within a sample nucleotide sequence. By changing such methylation-difference values, the bias-adjusted-methylation-assay system can change values indicating a presence or absence of a particular cancer, neurological disorder, or other disease that differs from initial methylation-difference values that come from initial (and uncorrected) methylation-level values.

In addition to improved accuracy and recovered biological signals, the bias-adjusted-methylation-assay system can improve the computational speed with which a methylation-assay-correction model determines a corrected methylation-level value. As noted above, some existing methylation-assay-correction models expend considerable time and computer processing to remove, from a sample's data, nucleotide reads comprising cytosines that bisulfite (or other enzyme) failed or incompletely converted into uracil. Further, based on a recently filed patent application by Illumina, Inc. and Illumina Cambridge Limited, some sequencing systems could use a specialized convolutional neural network (or other machine-learning model) to determine factors or scores indicating an error level with which a given methylation sequencing assay detects methylation of cytosine bases, as described by Machine-Learning Models for Detecting and Adjusting Values for Nucleotide Methylation Levels, Provisional U.S. Application No. 63/268,550 (filed Feb. 25, 2022). Unlike some existing methylation-assay-correction models, such as the read filter in Bismark Bisulfite Mapper, the bias-adjusted-methylation-assay system does not need to waste computing resources to analyze and remove individual nucleotide reads with failed or incomplete conversion of cytosine bases.
Further, unlike a neural network that can take minutes to hours to process data representing nucleotide sequences as a basis for adjusting or correcting methylation-level values, the bias-adjusted-methylation-assay system can execute its computational model in less than a second to determine a corrected methylation-level value for an individual target cytosine base. The bias-adjusted-methylation-assay system, therefore, expedites the computational speed of determining a corrected methylation-level value in part by avoiding individual read filtering and the computer-processing time of a neural network.

In part due to more accurate methylation-level values, in certain implementations, the bias-adjusted-methylation-assay system also improves the computing efficiency and processing time consumed by specialized sequencing devices and/or computing devices running analysis software that perform methylation sequencing assays. As noted above, some existing sequencing systems re-run methylation sequencing assays on multiple samples or run different types of methylation sequencing assays to detect cytosine methylation more reliably. Rather than perform redundant or time-intensive processing on specialized sequencing devices, the bias-adjusted-methylation-assay system can execute a computationally efficient model to determine corrected methylation-level values for a specific sample nucleotide sequence analyzed by a given methylation sequencing assay-thereby obviating methylation-assay re-runs or diversified methylation-assay types. By leveraging estimated false positive and false negative rates and predicting numbers of nucleotide reads supporting unmethylated and methylated cytosine sites, the bias-adjusted-methylation-assay system can determine corrected methylation-level values that adjust for biases caused by the chemical unpredictability, imaging inaccuracies, or other failures of existing methylation sequencing assays.

Beyond improved computing efficiency, in some embodiments, the bias-adjusted-methylation-assay system also introduces a computational model that increases the flexibility with which a corrected methylation-level value can be applied to (or determined for) different organisms or methylation sequencing assays. For instance, some existing methylation-assay-correction models, such as the read filter for Bismark Bisulfite Mapper, are limited to specific enzyme-based methylation sequencing assays (e.g., bisulfite-based methylation sequencing assays) and/or methylation sequencing assays that convert cytosine bases at CpG sites for samples from mammals or similar organisms. By contrast, the bias-adjusted-methylation-assay system can perform a new computation model that determines corrected methylation-level values for (i) sample nucleotide sequences for different enzyme-based methylation sequencing assays and/or (ii) sample nucleotide sequences extracted from any organism with cytosine bases flanked by any contextual sequence, not merely CpG sites. Indeed, in some embodiments, the bias-adjusted-methylation-assay system determines a corrected methylation-level value for a target cytosine base from a sample nucleotide sequence extracted from a non-human organism.

Similarly, the bias-adjusted-methylation-assay system also introduces a computational model that increases the flexibility with which corrected methylation-level values can be interpreted in terms of contributing factors to improved methylation-level values. As noted above, new neural networks or other machine-learning models developed by Illumina, Inc. and Illumina Cambridge Limited can determine factors or scores indicating an error level with which a given methylation sequencing assay detects methylation of cytosine bases. But a deep neural network leveraged to correct methylation-level values could transform and manipulate sequence data (or other input data) many times over, changing from one uninterpretable latent vector to another such latent vector across the various layers and neurons. In many cases, the internal data of such deep neural networks is uninterpretable and impossible to utilize in any way outside of the neural network architecture itself. By contrast, the bias-adjusted-methylation-assay system introduces a computational model in which discernable factors-such as estimated false positive rate, estimated false negative rate, corrected numbers of nucleotide reads supporting methylated or unmethylated cytosine sites-can be quickly determined and analyzed in terms of the degree to which an individual factor impacts a corrected methylation-level value.

As illustrated by the foregoing discussion, the present disclosure utilizes a variety of terms to describe features and advantages of the bias-adjusted-methylation-assay system. As used herein, for example, the term "methylation sequencing assay" refers to an assay that detects, measures, or quantifies methylation of cytosine from an oligonucleotide or other nucleotide sequence. In some cases, a methylation sequencing assay detects or quantifies methylation of cytosine at particular target genomic regions or in particular cell types. As suggested above and explained below, some methylation sequencing assays quantify methylation in terms of methylation-level values.

Relatedly, the term "methylation-level value" refers to a numeric value indicating an amount, percentage, ratio, or quantity of cytosine to which a methyl group or hydroxymethyl group has been added or bonded. For instance, a methylation-level value includes a score (e.g., ranging from 0 to 1) that indicates a percentage or ratio of cytosine bases (e.g., at CpG or other cytosine sites) for particular genomic coordinates or genomic regions to which a methyl group has been added. In some cases, a methylation-level value is expressed as a beta value or an M value. To illustrate, a beta value may estimate a methylation level using a ratio of signal intensities between methylated alleles corresponding to a genomic coordinate and unmethylated alleles corresponding to the genomic coordinate, where 0 represents completely unmethylated and 1 represents completely methylated. By contrast, an M value may represent a log2 ratio of signal intensities of a methylated probe and an unmethylated probe corresponding to a cytosine base.

Relatedly, the term "corrected methylation-level value" refers to a numeric value that has been generated or adjusted to indicate an amount, percentage, ratio, or quantity of cytosine to which a methyl group or hydroxymethyl group has been added or bonded and account for a bias or error of a given methylation sequencing assay. For instance, a corrected methylation-level value includes a score (e.g., ranging from 0 to 1)-generated or adjusted to account for a given methylation sequencing assay's bias or error-to indicate a percentage or ratio of cytosine bases for particular genomic coordinates or genomic regions to which a methyl group has been added. Accordingly, a corrected methylation-level value includes a corrected beta value or a corrected M value.

As further used herein, the term "sample nucleotide sequence" refers to a sequence of nucleotides isolated or extracted from a sample organism (or a copy of such an isolated or extracted sequence). In particular, a sample nucleotide sequence includes a segment of a nucleic acid polymer that is isolated or extracted from a sample organism and composed of nitrogenous heterocyclic bases. For example, a sample nucleotide sequence can include a segment of deoxyribonucleic acid (DNA), ribonucleic acid (RNA), or other polymeric forms of nucleic acids or chimeric or hybrid forms of nucleic acids noted below. More specifically, in some cases, the sample nucleotide sequence is found in a sample prepared or isolated by a kit and received by a sequencing device.

As indicated above and as used herein, the term "false positive rate" refers to a rate at which a methylation sequencing assay incorrectly converts one or more cytosine bases within a nucleotide sequence into one or more other nucleobases. In particular, a false positive rate includes a rate at which a methylation sequencing assay incorrectly converts one or more unmethylated cytosine bases within a nucleotide sequence into one or more uracil bases or thymine bases.

Relatedly, as used herein, the term "false negative rate" refers to a rate at which a methylation sequencing assay fails to convert one or more cytosine bases within a nucleotide sequence into one or more other nucleobases. In particular, a false positive rate includes a rate at which a methylation sequencing assay fails to convert one or more methylated cytosine bases within a nucleotide sequence into one or more uracil bases or thymine bases.

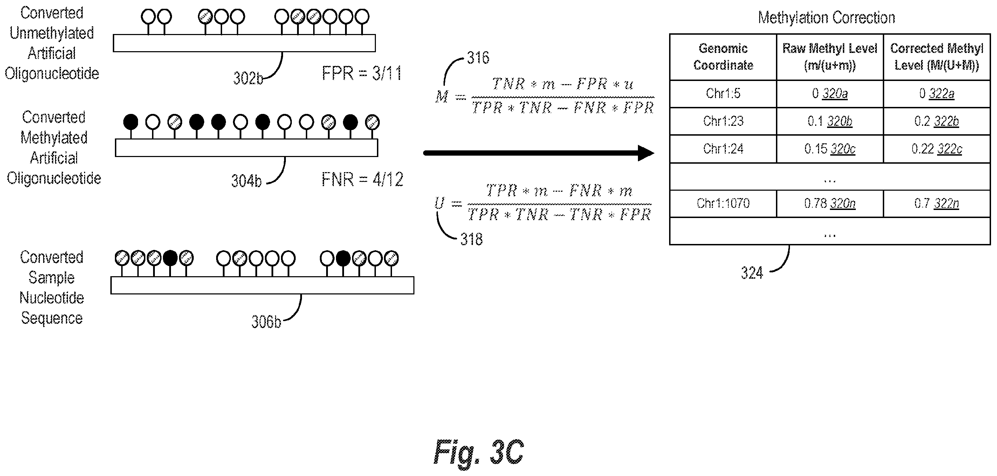

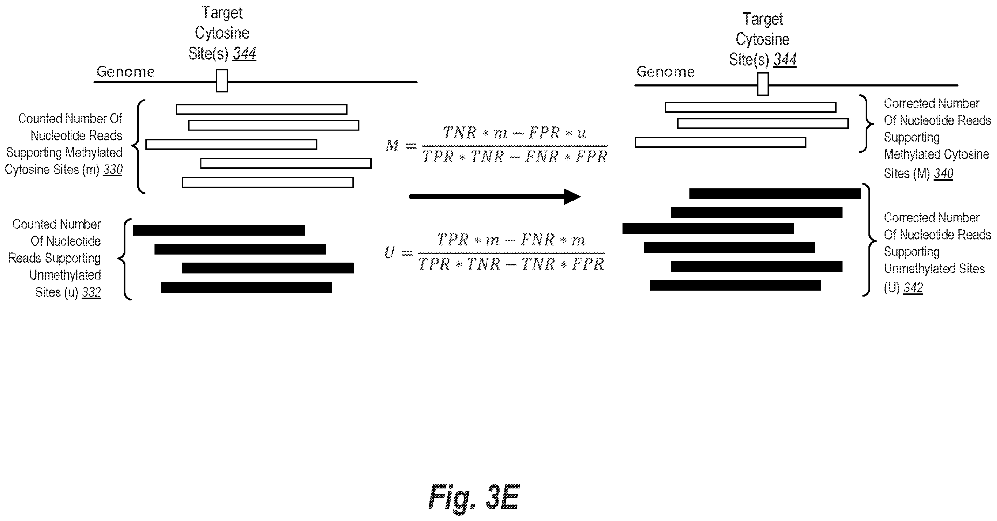

As further used herein, the term "corrected number of nucleotide reads" refers to a number of nucleotide reads predicted to support a methylated cytosine site or an unmethylated cytosine site within a sample nucleotide sequence. For example, in some cases, a first corrected number of nucleotide reads includes a number of nucleotide reads predicted to include a nucleobase (e.g., a uracil base or a thymine base) as evidence of a methylated cytosine site within a sample nucleotide sequence based on (i) a false positive rate and false negative rate at which a given methylation sequencing assay converts cytosine bases and (ii) a first and second counted number of nucleotide reads, from data generated by the given methylation sequencing assay, supporting methylated and unmethylated cytosine sites, respectively. As a further example, a second corrected number of nucleotide reads includes a number of nucleotide reads predicted to include a nucleobase (e.g., a cytosine base) as evidence of an unmethylated cytosine site within a sample nucleotide sequence based on (i) a false positive rate and false negative rate at which a given methylation sequencing assay converts cytosine bases and (ii) a first and second counted number of nucleotide reads, from data generated by the given methylation sequencing assay, supporting methylated and unmethylated cytosine sites, respectively. Accordingly, a corrected number of nucleotide reads does not necessarily represent a ground-truth number or a correct number of nucleotide reads supporting a methylated or unmethylated cytosine site, but rather a number that has either been adjusted or confirmed to be accurate as predicted by a computational model based on a false positive rate and a false negative rate. This disclosure provides further examples of corrected numbers of nucleotide reads below with respect to FIGS. 3C and 3E.

As further used herein, the term "artificial oligonucleotide" refers to a synthetically constructed or designed nucleotide sequence. In particular, an artificial oligonucleotide includes a nucleotide sequence that has been constructed or designed to include a particular number of methylated cytosine bases or a particular number of unmethylated cytosine bases. For instance, an artificial oligonucleotide includes an unmethylated spike-in oligonucleotide comprising a known number of unmethylated cytosine sites or a methylated spike-in oligonucleotide comprising a known number of methylated cytosine sites.

As further used herein, the term "contextual sequence" refers to a series of nucleobases from a nucleotide sequence that surround (e.g., flank on each side or neighbor) a target cytosine base or a target cytosine-guanine-dinucleotide pair at a CpG site. In some examples, a contextual sequence refers to a series of upstream and downstream nucleobases from a nucleotide sequence (e.g., sample nucleotide sequence or artificial oligonucleotide) that flank or surround a target cytosine base or a target cytosine-guanine-dinucleotide pair. Accordingly, a contextual sequence includes nucleobases from a nucleotide sequence that are located both upstream and downstream from genomic coordinate(s) or position(s) for a target cytosine base or a target cytosine-guanine-dinucleotide pair. Further, a contextual sequence may include the five, ten, fifteen, or other threshold number of nucleobases upstream of a target cytosine base and the five, ten, or fifteen or other threshold number of nucleobases downstream from the target cytosine. While the number of nucleobases upstream and downstream from a target cytosine base or from a target cytosine-guanine-dinucleotide pair may be equal to each other (e.g., 15 upstream nucleobases and 15 downstream nucleobases), in some embodiments, a contextual sequence includes numbers of nucleobases upstream and downstream from the target cytosine base or from the target cytosine-guanine-dinucleotide pair that do not equal each other (e.g., 14 upstream nucleobases and 15 downstream nucleobases).

As further used herein, the term "nucleotide read" (or simply "read") refers to an inferred sequence of one or more nucleobases (or nucleobase pairs) from all or part of a sample nucleotide sequence (e.g., a sample genomic sequence, complementary DNA). In particular, a nucleotide read includes a determined or predicted sequence of nucleobase calls for a nucleotide sequence (or group of monoclonal nucleotide sequences) from a sample library fragment corresponding to a genomic sample. For example, in some cases, a sequencing device determines a nucleotide read by generating nucleobase calls for nucleobases passed through a nanopore of a nucleotide-sample slide, determined via fluorescent tagging, or determined from a cluster in a flow cell.

As further used herein, the term "nucleobase call" (or simply "base call") refers to a determination or prediction of a particular nucleobase (or nucleobase pair) for an oligonucleotide (e.g., read) during a sequencing cycle or for a genomic coordinate of a sample genome. In particular, a nucleobase call can indicate (i) a determination or prediction of the type of nucleobase that has been incorporated within an oligonucleotide on a nucleotide-sample slide (e.g., read-based nucleobase calls) or (ii) a determination or prediction of the type of nucleobase that is present at a genomic coordinate or region within a genome, including a variant call or a non-variant call in a digital output file. In some cases, for a nucleotide read, a nucleobase call includes a determination or a prediction of a nucleobase based on intensity values resulting from fluorescent-tagged nucleotides added to an oligonucleotide of a nucleotide-sample slide (e.g., in a cluster of a flow cell). Alternatively, a nucleobase call includes a determination or a prediction of a nucleobase from chromatogram peaks or electrical current changes resulting from nucleotides passing through a nanopore of a nucleotide-sample slide. By contrast, a nucleobase call can also include a final prediction of a nucleobase at a genomic coordinate of a sample genome for a variant call file (VCF) or other base-call-output file-based on nucleotide reads corresponding to the genomic coordinate. Accordingly, a nucleobase call can include a base call corresponding to a genomic coordinate and a reference genome, such as an indication of a variant or a non-variant at a particular location corresponding to the reference genome. Indeed, a nucleobase call can refer to a variant call, including but not limited to, a single nucleotide variant (SNV), an insertion or a deletion (indel), or base call that is part of a structural variant. As suggested above, a single nucleobase call can be an adenine (A) call, a cytosine (C) call, a guanine (G) call, or a thymine (T) call.

As further used herein, the term "genomic coordinate" (or sometimes simply "coordinate") refers to a particular location or position of a nucleobase within a genome (e.g., an organism's genome or a reference genome). In some cases, a genomic coordinate includes an identifier for a particular chromosome of a genome and an identifier for a position of a nucleobase within the particular chromosome. For instance, a genomic coordinate or coordinates may include a number, name, or other identifier for a chromosome (e.g., chr1 or chrX) and a particular position or positions, such as numbered positions following the identifier for a chromosome (e.g., chr1:1234570 or chr1:1234570-1234870). Further, in certain implementations, a genomic coordinate refers to a source of a reference genome (e.g., mt for a mitochondrial DNA reference genome or SARS-CoV-2 for a reference genome for the SARS-CoV-2 virus) and a position of a nucleobase within the source for the reference genome (e.g., mt:16568 or SARS-CoV-2:29001). By contrast, in certain cases, a genomic coordinate refers to a position of a nucleobase within a reference genome without reference to a chromosome or source (e.g., 29727).

As mentioned above, a "genomic region" refers to a range of genomic coordinates. Like genomic coordinates, in certain embodiments, a genomic region may be identified by an identifier for a chromosome and a particular position or positions, such as numbered positions following the identifier for a chromosome (e.g., chr1:1234570-1234870).

The following paragraphs describe the bias-adjusted-methylation-assay system with respect to illustrative figures that portray example embodiments and implementations. For example, FIG. 1 illustrates a schematic diagram of a computing system 100 in which a bias-adjusted-methylation-assay system 106 operates in accordance with one or more embodiments. As illustrated, the computing system 100 includes server device(s) 102, a sequencing device 114, and a user client device 110 connected via a network 118. While FIG. 1 shows an embodiment of the bias-adjusted-methylation-assay system 106, this disclosure describes alternative embodiments and configurations below. As shown in FIG. 1, the sequencing device 114, the server device(s) 102, and the user client device 110 can communicate with each other via the network 118. The network 118 comprises any suitable network over which computing devices can communicate. Example networks are discussed in additional detail below with respect to FIG. 8.

As indicated by FIG. 1, the sequencing device 114 comprises a sequencing device system 116 for sequencing a genomic sample or other nucleic-acid polymer, such as when sequencing oligonucleotides extracted from a genomic sample as part of a methylation sequencing assay. In some embodiments, by executing the sequencing device system 116, the sequencing device 114 analyzes nucleotide sequences or oligonucleotides extracted from genomic samples to generate nucleotide reads or other data utilizing computer implemented methods and systems (described herein) either directly or indirectly on the sequencing device 114. More particularly, the sequencing device 114 receives nucleotide-sample slides (e.g., flow cells) comprising nucleotide sequences extracted from samples and then copies and determines the nucleobase sequence of such extracted nucleotide sequences. As part of a methylation sequencing assay, for instance, the sequencing device 114 may determine nucleobase calls for nucleotide reads comprising CpG sites or other cytosine sites.

As suggested above, by executing the sequencing device system 116, the sequencing device 114 can run one or more sequencing cycles as part of a sequencing run. By executing the bias-adjusted-methylation-assay system 106, for instance, the sequencing device 114 can (i) sequence certain uracil bases that were converted from methylated cytosine bases and that are part of a nucleotide read and (ii) determine nucleobase calls of thymine for such uracil bases as part of a methylation sequencing assay. In one or more embodiments, the sequencing device 114 utilizes Sequencing by Synthesis (SBS) to sequence nucleic-acid polymers into nucleotide reads.

In some cases, the server device(s) 102 is located at or near a same physical location of the sequencing device 114 or remotely from the sequencing device 114. Indeed, in some embodiments, the server device(s) 102 and the sequencing device 114 are integrated into a same computing device. The server device(s) 102 may run a sequencing system 104 or the bias-adjusted-methylation-assay system 106 to generate, receive, analyze, store, and transmit digital data, such as by receiving base-call data or determining variant calls based on analyzing such base-call data.

As further suggested by FIG. 1, the sequencing device 114 may send (and the server device(s) 102 may receive) base-call data generated during a sequencing run of the sequencing device 114. By executing software in the form of the sequencing system 104 or the bias-adjusted-methylation-assay system 106, the server device(s) 102 may align nucleotide reads with a reference genome and determine variant calls based on the aligned nucleotide reads. The server device(s) 102 may also communicate with the user client device 110. In particular, the server device(s) 102 can send data to the user client device 110, including a variant call file (VCF), or other information indicating nucleobase calls, sequencing metrics, error data, or other metrics.

In some embodiments, the server device(s) 102 comprise a distributed collection of servers where the server device(s) 102 include a number of server devices distributed across the network 118 and located in the same or different physical locations. Further, the server device(s) 102 can comprise a content server, an application server, a communication server, a web-hosting server, or another type of server.

As further illustrated and indicated in FIG. 1, the user client device 110 can generate, store, receive, and send digital data. In particular, the user client device 110 can receive variant calls and corresponding sequencing metrics from the server device(s) 102 or receive base-call data (e.g., binary base call (BCL) file or fast-all quality (FASTQ) file) and corresponding sequencing metrics from the sequencing device 114. Furthermore, the user client device 110 may communicate with the server device(s) 102 to receive a VCF comprising nucleobase calls and/or other metrics, such as base-call-quality metrics or pass-filter metrics. The user client device 110 can accordingly present or display information pertaining to variant calls or other nucleobase calls within a graphical user interface to a user associated with the user client device 110. In particular, the user client device 110 can present results from a methylation sequencing assay or graphics that indicate either or both of methylation-level values and corrected methylation-level values for target cytosine bases.

Although FIG. 1 depicts the user client device 110 as a desktop or laptop computer, the user client device 110 may comprise various types of client devices. For example, in some embodiments, the user client device 110 includes non-mobile devices, such as desktop computers or servers, or other types of client devices. In yet other embodiments, the user client device 110 includes mobile devices, such as laptops, tablets, mobile telephones, or smartphones. Additional details regarding the user client device 110 are discussed below with respect to FIG. 8.

As further illustrated in FIG. 1, the user client device 110 includes a sequencing application 112. The sequencing application 112 may be a web application or a native application stored and executed on the user client device 110 (e.g., a mobile application, desktop application). The sequencing application 112 can include instructions that (when executed) cause the user client device 110 to receive data from the bias-adjusted-methylation-assay system 106 and present, for display at the user client device 110, base-call data (e.g., from a BCL), data from a VCF, or data from a methylation sequencing assay.

As further illustrated in FIG. 1, a version of the bias-adjusted-methylation-assay system 106 may be located on the user client device 110 as part of the sequencing application 112 or on the sequencing device 114. Accordingly, in some embodiments, the bias-adjusted-methylation-assay system 106 is implemented by (e.g., located entirely or in part) on the user client device 110. In yet other embodiments, the bias-adjusted-methylation-assay system 106 is implemented by one or more other components of the computing system 100, such as the sequencing device 114. In particular, the bias-adjusted-methylation-assay system 106 can be implemented in a variety of different ways across the sequencing device 114, the user client device 110, and the server device(s) 102. For example, the bias-adjusted-methylation-assay system 106 can be downloaded from the server device(s) 102 to the sequencing device 114 and/or the user client device 110 where all or part of the functionality of the bias-adjusted-methylation-assay system 106 is performed at each respective device within the computing system.

As indicated above, the bias-adjusted-methylation-assay system 106 can use a computationally efficient model to determine corrected methylation-level values for cytosine bases within specific sample nucleotide sequences analyzed by a given methylation sequencing assay. In accordance with one or more embodiments, FIG. 2 illustrates an example of the bias-adjusted-methylation-assay system 106 both identifying an initial methylation-level value for a target cytosine base within a sample nucleotide sequence and determining a corrected methylation-level value for the target cytosine base. As an overview of generating a such corrected value, as shown in FIG. 2, the bias-adjusted-methylation-assay system 106 (i) determines a false positive rate and a false negative rate at which a methylation sequencing assay converts cytosine bases, (ii) predicts corrected numbers of nucleotide reads supporting methylated and unmethylated cytosine sites within the sample nucleotide sequence based on the false positive rate and the false negative rate, and (iii) determines the corrected methylation-level value for the target cytosine base based on the predicted corrected numbers of nucleotide reads.

As shown in FIG. 2, for instance, the bias-adjusted-methylation-assay system 106 identifies methylation-level value(s) 210 for cytosine base(s) 200 within a sample nucleotide sequence 204 determined by a methylation sequencing assay 202 by either (i) accessing or receiving the methylation-level value(s) 210 from a computing device or (ii) determining the methylation-level value(s) 210 for the cytosine base(s) 200 using the methylation sequencing assay 202. For example, in some cases, the bias-adjusted-methylation-assay system 106 inputs or runs the sample nucleotide sequence 204 through the methylation sequencing assay 202, such as TAPS and/or an APOBEC-based assay. In particular, in certain embodiments, the bias-adjusted-methylation-assay system 106 performs a methylation sequencing assay or identifies methylation-level values from a methylation sequencing assay that uses APOBEC3A as an enzyme to deaminate cytosine, as described by Altered Cytidine Deaminases and Methods of Use, U.S. Provisional Application No. 63/328,444 (filed Apr. 7, 2022).

As just indicated, the sample nucleotide sequence 204 comprises one or more cytosine bases. As depicted in FIG. 2, for instance, the sample nucleotide sequence 204 includes fifteen cytosine bases, including both methylated and unmethylated cytosine bases. As depicted by FIG. 2, the open circles of the sample nucleotide sequence 204 represent six unmethylated cytosine bases of the cytosine base(s) 200, and the dark-filled or stripe-patterned circles of the sample nucleotide sequence 204 represent nine methylated cytosine bases. In this example, the dark-filled and stripe-patterned circles of the sample nucleotide sequence 204 represent different degrees of methylation; but each of the dark-filled and stripe-patterned circles represent a methylated cytosine base of the cytosine base(s) 200. Although not depicted in FIG. 2, in some embodiments, a sample nucleotide sequence includes methylated cytosine bases of a same degree of methylation.

In certain cases, the sample nucleotide sequence 204 constitutes a sample library fragment with genomic DNA from a sample comprising the cytosine base(s) 200. Consistent with the disclosure above, in certain implementations, the bias-adjusted-methylation-assay system 106 uses a TET enzyme, APOBEC3A enzyme, and/or a different enzyme to convert the cytosine bases 204a-204n to uracil bases as part of the methylation sequencing assay 202.

As further part of the methylation sequencing assay 202, in some embodiments, the bias-adjusted-methylation-assay system 106 amplifies and determines nucleobase calls for the sample nucleotide sequence 204 and complementary strands using the sequencing device 114. In some such cases, the bias-adjusted-methylation-assay system 106 uses SBS to determine nucleobase calls for the sample nucleotide sequence 204 when sequencing or amplifying a nucleotide read of nucleotide reads 206, including thymine nucleobase calls for one or more of the cytosine base(s) 200 that have been converted into uracil bases or thymine bases. Along with other determined nucleotide reads, in some cases, the sequencing device 114 sends base-call data to the server device(s) 102. As further indicated by FIG. 2, in certain implementations, the bias-adjusted-methylation-assay system 106 uses the server device(s) 102 to align the nucleotide reads 206 with a reference genome 208 or the non-enzymatically converted nucleotide reads and determine variant calls. As part of the methylation sequencing assay 202, for instance, the bias-adjusted-methylation-assay system 106 identifies thymine bases corresponding to the cytosine base(s) 200 that vary from cytosine bases at cytosine sites within the reference genome 208 or within the non-enzymatically converted nucleotide reads.

As further shown in FIG. 2, the bias-adjusted-methylation-assay system 106 determines the methylation-level value(s) 210 for the cytosine base(s) 200 as part of the methylation sequencing assay 202. For instance, in some cases, the bias-adjusted-methylation-assay system 106 determines beta value(s) each indicating a percentage or ratio of the nucleotide reads 206 covering the cytosine base(s) 200 to which a methyl group or hydroxymethyl group has been added. In particular, the beta value may estimate a methylation level using a ratio of signal intensities between methylated alleles corresponding to a genomic coordinate for a cytosine base and unmethylated alleles corresponding to the genomic coordinate for the cytosine base. Alternatively, the methylation-level value(s) 210 may each constitute an M value that indicates a log2 ratio of signal intensities of a methylated probe corresponding to a cytosine base and an unmethylated probe corresponding to the cytosine base.

In addition to determining or otherwise identifying the methylation-level value(s) 210 for the cytosine base(s) 200, as further shown in FIG. 2, the bias-adjusted-methylation-assay system 106 determines corrected methylation-level value(s) 228 for the cytosine base(s) 200 within the sample nucleotide sequence 204. To determine such corrected value(s), the bias-adjusted-methylation-assay system 106 determines a false positive rate 216 and a false negative rate 218 at which the methylation sequencing assay 202 converts cytosine bases within nucleotide sequences. To determine such rates, in some embodiments, the bias-adjusted-methylation-assay system 106 identifies a predetermined false positive rate and false negative rate for the methylation sequencing assay 202. Alternatively, as explained further below, the bias-adjusted-methylation-assay system 106 determines the false positive rate 216 and the false negative rate 218 by running artificial oligonucleotides 212 and 214 with known methylated and unmethylated cytosine bases through the methylation sequencing assay 202. By running the artificial oligonucleotides 212 and 214 through the methylation sequencing assay 202, the bias-adjusted-methylation-assay system 106 determines numbers of methylated and unmethylated cytosine bases that the methylation sequencing assay 202 correctly or incorrectly converts into uracil or thymine bases. From the number of correctly or incorrectly converted cytosine bases, the bias-adjusted-methylation-assay system 106 can determine the false positive rate 216 and the false negative rate 218.

As further shown in FIG. 2, in some embodiments, the bias-adjusted-methylation-assay system 106 further identifies, from data generated by the methylation sequencing assay 202, a counted number of nucleotide reads 220 supporting methylated cytosine sites within the sample nucleotide sequence 204 and a counted number of nucleotide reads 222 supporting unmethylated cytosine sites within the sample nucleotide sequence 204. For instance, the bias-adjusted-methylation-assay system 106 identifies a first counted number and a second counted number of nucleotide reads supporting methylated and unmethylated cytosine sites, respectively, from a cytosine report file or based on an alignment between the nucleotide reads 206 and the reference genome 208. As described further below, the first counted number of nucleotide reads and the second counted number of nucleotide reads may be specific to methylated and unmethylated cytosine bases at particular genomic coordinates.

Based on such rates and counted numbers of nucleotide reads, as further shown in FIG. 2, the bias-adjusted-methylation-assay system 106 predicts a corrected number of nucleotide reads 224 supporting methylated cytosine sites within the sample nucleotide sequence 204 and a corrected number of nucleotide reads 226 supporting methylated cytosine sites within the sample nucleotide sequence 204. As further described below with respect to FIGS. 3C and 3E, for example, the bias-adjusted-methylation-assay system 106 applies a computational model to derive both the corrected number of nucleotide reads 224 and the corrected number of nucleotide reads 226 from the false positive rate 216, the false negative rate 218, the counted number of nucleotide reads 220, and the counted number of nucleotide reads 222.

As further shown in FIG. 2, the bias-adjusted-methylation-assay system determines the corrected methylation-level value(s) 228 for the cytosine base(s) 200 within the sample nucleotide sequence 204-based on the corrected number of nucleotide reads 224 supporting methylated cytosine sites and the corrected number of nucleotide reads 226 supporting methylated cytosine sites. In some embodiments, for instance, the bias-adjusted-methylation-assay system 106 determines, as the corrected number of nucleotide reads 224, a quotient of the corrected number of nucleotide reads 224 supporting methylated cytosine sites (M) over a sum of the corrected number of nucleotide reads 226 supporting methylated cytosine sites and the corrected number of nucleotide reads 224 supporting methylated cytosine sites (U+M).

Having determined both the methylation-level value(s) 210 and the corrected methylation-level value(s) 228, the bias-adjusted-methylation-assay system 106 can provide both such values to a computing device. As shown in FIG. 2, for instance, the bias-adjusted-methylation-assay system 106 provides data to a computing device 230 to display the methylation-level value(s) 210 and the corrected methylation-level value(s) 228 for the sample nucleotide sequence 204 within a graphical user interface. Because the computational model is transparent and the factors underlying the corrected methylation-level value(s) 228 known, recipients of the corrected methylation-level value(s) 228 can choose a degree to which they rely on the corrected methylation-level value(s) 228.

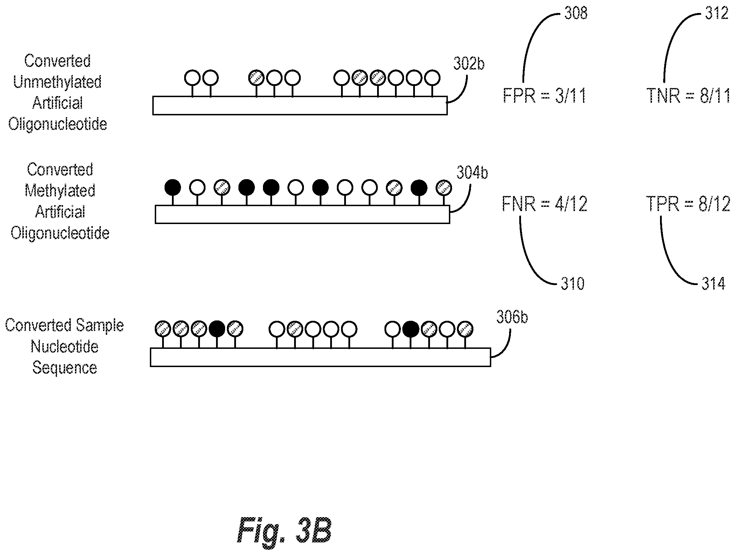

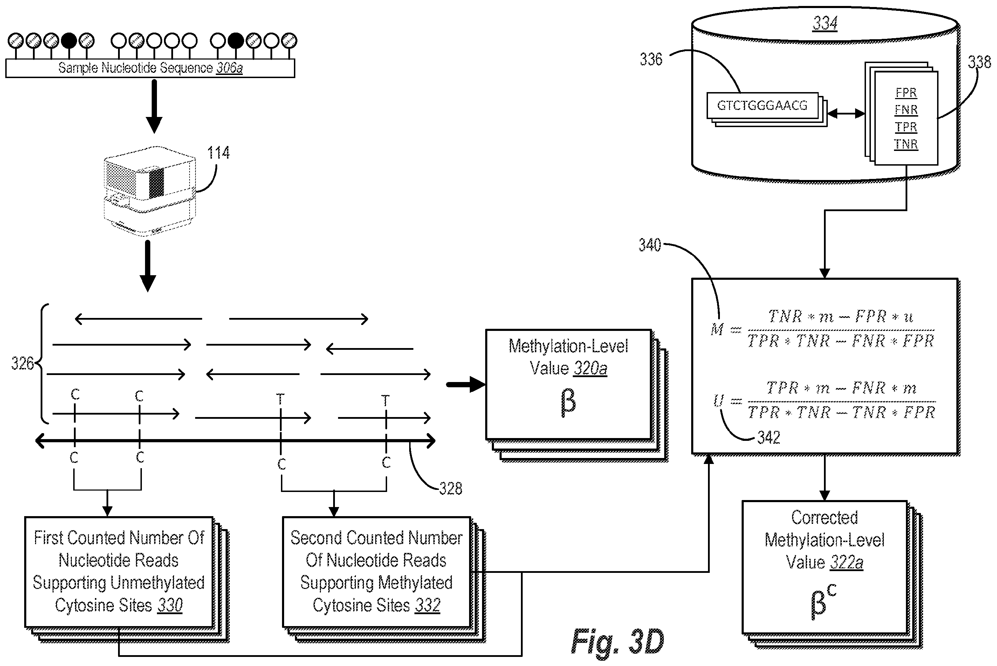

As suggested above, the bias-adjusted-methylation-assay system 106 can determine corrected methylation-level values specific to both a given methylation sequencing assay and specific to target cytosine bases within sample nucleotide sequences. In accordance with one or more embodiments, FIGS. 3A-3E illustrate the bias-adjusted-methylation-assay system 106 determining corrected methylation-level values for specific, target cytosine bases. For instance, FIG. 3A depicts the bias-adjusted-methylation-assay system 106 running unmethylated artificial oligonucleotides, methylated artificial oligonucleotides, and sample nucleotide sequences through a methylation sequencing assay. FIG. 3B depicts the bias-adjusted-methylation-assay system 106 determining a false positive rate and a false negative rate at which the methylation sequencing assay converts cytosine bases into uracil bases or thymine bases. FIG. 3C depicts the bias-adjusted-methylation-assay system 106 determining corrected methylation-level values for target cytosine bases at specific genomic coordinates based on the false positive rate and the false negative rate. FIG. 3D illustrates the bias-adjusted-methylation-assay system 106 (i) identifying predetermined false positive rates and false negative rates specific to a contextual sequence for a target cytosine base and (ii) determining a corrected methylation-level value specific to the contextual sequence flanking the target cytosine base. FIG. 3E illustrates the bias-adjusted-methylation-assay system 106 applying a computational model to predict a first corrected number of nucleotide reads supporting methylated cytosine sites within a sample nucleotide sequence and a second corrected number of nucleotide reads supporting unmethylated cytosine sites within a sample nucleotide sequence.

To determine a false positive rate and a false negative rate for a methylation sequencing assay, the bias-adjusted-methylation-assay system 106 optionally uses unmethylated artificial oligonucleotides and methylated artificial oligonucleotides. As shown in FIG. 3A, for instance, the bias-adjusted-methylation-assay system 106 accesses or receives an unmethylated artificial oligonucleotide 302a comprising a known number of unmethylated cytosine bases (e.g., eleven unmethylated cytosine bases) and a methylated artificial oligonucleotide 304a comprising a known number of methylated cytosine bases (e.g., twelve methylated cytosine bases). As depicted by FIG. 3A, the open circles of the unmethylated artificial oligonucleotide 302a represent eleven known unmethylated cytosine bases, and the dark-filled or stripe-patterned circles of the methylated artificial oligonucleotide 304a represent twelve known methylated cytosine bases. In this example, the dark-filled and stripe-patterned circles of the methylated artificial oligonucleotide 304a represent different degrees of methylation; but each of the dark-filled and stripe-patterned circles represent a methylated cytosine base. In some cases, the unmethylated artificial oligonucleotide 302a and the methylated artificial oligonucleotide 304a each take the form of a spike-in oligonucleotide that has been prepared or designed with known numbers of unmethylated cytosine sites and methylated cytosine sites, respectively, to test the conversion accuracy of a methylation sequencing assay 300.

For illustrative purposes, the methylation sequencing assay 300 represented in FIGS. 3A-3E comprises an APOBEC enzyme that converts methylated cytosine bases into uracil or thymine bases, but does not by design convert unmethylated cytosine bases into uracil or thymine bases. In some embodiments, however, the bias-adjusted-methylation-assay system 106 determines corrected methylation-level values for methylation sequencing assays that convert unmethylated cytosine bases, but does not by design convert methylated cytosine bases.

In addition to optionally using the unmethylated artificial oligonucleotide 302a and the methylated artificial oligonucleotide 304a, the bias-adjusted-methylation-assay system 106 inputs a sample nucleotide sequence 306a through the methylation sequencing assay 300. As depicted by FIG. 3A, the open circles of the sample nucleotide sequence 306a represent eight unmethylated cytosine bases, and the dark-filled or stripe-patterned circles of the sample nucleotide sequence 306a represent seven methylated cytosine bases. In this example, the dark-filled and stripe-patterned circles of the sample nucleotide sequence 306a represent different degrees of methylation; but each of the dark-filled and stripe-patterned circles represent a methylated cytosine base. Although not depicted in FIGS. 3A-3D, in some embodiments, a sample nucleotide sequence includes methylated cytosine bases of a same degree of methylation within the sample nucleotide sequence and a methylated artificial oligonucleotide includes methylated cytosine bases of a same degree of methylation within the methylated artificial oligonucleotide.

As indicated above, in some cases, the sample nucleotide sequence 306a constitutes a segment of genomic DNA extracted or copied from a genomic sample and prepared with adapters as part of a sample library fragment for sequencing. By contrast, in certain cases, the sample nucleotide sequence 306a constitutes a segment of complementary DNA synthesized from DNA extracted or copied from a genomic sample. For sequencing purposes, although not depicted in FIG. 3A, the sample nucleotide sequence 306a can comprise adapters or primers.

As further shown in FIG. 3A, the bias-adjusted-methylation-assay system 106 runs the unmethylated artificial oligonucleotide 302a, the methylated artificial oligonucleotide 304a, and the sample nucleotide sequence 306a through the methylation sequencing assay 300. Consistent with the disclosure above, by running the methylation sequencing assay 300, the bias-adjusted-methylation-assay system 106 transforms the unmethylated artificial oligonucleotide 302a, the methylated artificial oligonucleotide 304a, and the sample nucleotide sequence 306a into a converted unmethylated artificial oligonucleotide 302b, a converted methylated artificial oligonucleotide 304b, and a converted sample nucleotide sequence 306b, respectively.

In FIG. 3A, a dark-filled or stripe-patterned circle-when within the converted unmethylated artificial oligonucleotide 302b, the converted methylated artificial oligonucleotide 304b, or the converted sample nucleotide sequence 306b-represents a uracil base or a thymine base converted from a cytosine base. Accordingly, as indicated by the change in shading or patterns within the circles representing cytosine bases to uracil or thymine bases, the methylation sequencing assay 300 (i) converts three of the eleven unmethylated cytosine bases within the unmethylated artificial oligonucleotide 302a into uracil or thymine bases and (ii) converts eight of the twelve methylated cytosine bases within the methylated artificial oligonucleotide 304a into uracil or thymine bases. By contrast, as also indicated by the change in shading or patterns within the circles representing cytosine bases in the sample nucleotide sequence 306a to uracil or thymine bases in the converted sample nucleotide sequence 306b, the bias-adjusted-methylation-assay system 106 converts nine of the fifteen cytosine bases within the sample nucleotide sequence 306a into uracil or thymine bases in the converted sample nucleotide sequence 306b.

As indicated above, in some cases, the bias-adjusted-methylation-assay system 106 selectively determines corrected methylation-level values for the methylation sequencing assay 300 (or a target cytosine base) depending on a number of nucleotide reads aligning with (and/or supporting a determination of a methylated or unmethylated cytosine base for) the target cytosine base. For instance, in some embodiments, the bias-adjusted-methylation-assay system 106 determines a counted number of nucleotide reads aligning with (or covering) a genomic coordinate within a reference genome corresponding to a target cytosine base. Alternatively, in some embodiments, the bias-adjusted-methylation-assay system 106 determines a counted number of nucleotide reads supporting a determination of a methylated cytosine base or a counted number of nucleotide reads supporting a determination of an unmethylated cytosine base at a genomic coordinate of a target cytosine base.

If the counted number of nucleotide reads (i) covering the genomic coordinate corresponding to the target cytosine base or (ii) having a nucleobase supporting a determination of a methylated cytosine or an unmethylated cytosine base satisfies a coverage threshold, in some embodiments, in some cases, the bias-adjusted-methylation-assay system 106 proceeds with a computational model and generates a corrected methylation-level value for the target cytosine base. Such a coverage threshold may be, for example, twenty, thirty, forty, or fifty nucleotide reads that (i) align with or cover the genomic coordinate corresponding to the target cytosine or (ii) include a nucleobase supporting a determination of a methylated cytosine or an unmethylated cytosine base for the target cytosine base. But the bias-adjusted-methylation-assay system 106 may use any threshold number of counted nucleotide reads as a coverage threshold. If, however, the counted number of nucleotide reads (i) covering the genomic coordinate corresponding to the target cytosine base or (ii) having a nucleobase supporting a determination of a methylated cytosine or an unmethylated cytosine base fails to satisfy the coverage threshold, in some embodiments, the bias-adjusted-methylation-assay system 106 does not proceed with the computational model and does not generate a corrected methylation-level value for the target cytosine base.

Because the methylation sequencing assay 300 comprises enzymes that selectively convert methylated cytosine bases into uracil bases, but not unmethylated cytosine bases into uracil bases by design, the methylation sequencing assay 300 is expected to convert methylated cytosine bases of the methylated artificial oligonucleotide 304a, and not the unmethylated cytosine bases of the unmethylated artificial oligonucleotide 302a. But the APOBEC enzyme for the methylation sequencing assay 300 sometimes fails to completely convert methylated cytosine bases and sometimes converts unmethylated cytosine bases contrary to the assay design. As shown in FIG. 3B, the bias-adjusted-methylation-assay system 106 can optionally leverage such failed conversions and unexpected conversions to determine a false positive rate 308 and a false negative rate 310 at which the methylation sequencing assay 300 converts cytosine bases within nucleotide sequences.

In particular, the bias-adjusted-methylation-assay system 106 determines the false positive rate 308 and the false negative rate 310 for the methylation sequencing assay 300 based on expected and actual conversions (i) between the unmethylated artificial oligonucleotide 302a and the converted unmethylated artificial oligonucleotide 302b and (ii) between the methylated artificial oligonucleotide 304a and the converted methylated artificial oligonucleotide 304b. As shown in FIG. 3B, for instance, the bias-adjusted-methylation-assay system 106 determines that the methylation sequencing assay 300 incorrectly converts three of the eleven unmethylated cytosine bases from the unmethylated artificial oligonucleotide 302a into uracil or thymine bases in the converted unmethylated artificial oligonucleotide 302b. By determining a quotient of the number of converted unmethylated cytosine bases (e.g., three) within the converted unmethylated artificial oligonucleotide 302b over a total number (e.g., eleven) of the unmethylated cytosine bases within the unmethylated artificial oligonucleotide 302a, in some embodiments, the bias-adjusted-methylation-assay system 106 determines the false positive rate 308 (e.g., three divided by eleven) at which the methylation sequencing assay 300 converts unmethylated cytosine bases.

Conversely, as further shown in FIG. 3B, the bias-adjusted-methylation-assay system 106 determines that the methylation sequencing assay 300 fails to convert four of the twelve methylated cytosine bases from the methylated artificial oligonucleotide 304a into uracil or thymine bases in the converted methylated artificial oligonucleotide 304b. By determining a quotient of the number of unconverted methylated cytosine bases (e.g., four) within the converted methylated artificial oligonucleotide 304b over a total number (e.g., twelve) of the methylated cytosine bases within the methylated artificial oligonucleotide 304a, the bias-adjusted-methylation-assay system 106 determines the false negative rate 310 (e.g., four divided by twelve) at which the methylation sequencing assay 300 converts methylated cytosine bases.

In addition to determining the false positive rate 308 and the false negative rate 310, in some embodiments, the bias-adjusted-methylation-assay system 106 similarly determines a true negative rate 312 and a true positive rate 314 for the methylation sequencing assay 300 based on expected and actual conversions (i) between the unmethylated artificial oligonucleotide 302a and the converted unmethylated artificial oligonucleotide 302b and (ii) between the methylated artificial oligonucleotide 304a and the converted methylated artificial oligonucleotide 304b. As shown in FIG. 3B, for instance, the bias-adjusted-methylation-assay system 106 determines that the methylation sequencing assay 300 does not convert eight of the eleven unmethylated cytosine bases from the unmethylated artificial oligonucleotide 302a into uracil or thymine bases in the converted unmethylated artificial oligonucleotide 302b. By determining a quotient of the number of non-converted unmethylated cytosine bases (e.g., eight) within the converted unmethylated artificial oligonucleotide 302b over a total number (e.g., eleven) of the unmethylated cytosine bases within the unmethylated artificial oligonucleotide 302a, the bias-adjusted-methylation-assay system 106 determines the true negative rate 312 (e.g., eight divided by eleven) at which the methylation sequencing assay 300 converts unmethylated cytosine bases.

Conversely, as further shown in FIG. 3B, the bias-adjusted-methylation-assay system 106 determines that the methylation sequencing assay 300 converts eight of the twelve methylated cytosine bases from the methylated artificial oligonucleotide 304a into uracil or thymine bases in the converted methylated artificial oligonucleotide 304b. By determining a quotient of the number of converted methylated cytosine bases (e.g., eight) within the converted methylated artificial oligonucleotide 304b over a total number (e.g., twelve) of the methylated cytosine bases within the methylated artificial oligonucleotide 304a, the bias-adjusted-methylation-assay system 106 determines the true positive rate 314 (e.g., eight divided by twelve) at which the methylation sequencing assay 300 converts methylated cytosine bases.

Based on the false positive rate 308, the false negative rate 310, the true negative rate 312, and the true positive rate 314, as shown in FIG. 3C, the bias-adjusted-methylation-assay system 106 predicts corrected numbers of nucleotide reads supporting methylated and unmethylated cytosine sites and determines corrected methylation-level values for target cytosine bases at specific genomic coordinates within the sample nucleotide sequence 306a. Based on such rates, in some embodiments, the bias-adjusted-methylation-assay system 106 likewise determines corrected methylation-level values for target cytosine bases at specific genomic coordinates within other sample nucleotide sequences from a genomic sample. Indeed, the bias-adjusted-methylation-assay system 106 can determine such corrected methylation-level values specific to cytosine bases at particular genomic coordinates and specific to the methylation sequencing assay 300.

In addition to the relevant rates, as indicated above, the bias-adjusted-methylation-assay system 106 can identify, from the methylation sequencing assay 300, counted numbers of nucleotide reads supporting a determination of methylated cytosine bases or unmethylated cytosine bases. For instance, the bias-adjusted-methylation-assay system 106 identifies, from a cytosine report file or other data generated by the methylation sequencing assay 300, (i) a first counted number of nucleotide reads supporting a methylated cytosine base at a particular genomic coordinate within the sample nucleotide sequence 306a and (ii) a second counted number of nucleotide reads supporting an unmethylated cytosine base at a particular genomic coordinate within the sample nucleotide sequence 306a. Such a cytosine report may include, for instance, a text file comprising counted numbers of nucleotide reads supporting particular cytosine bases at particular genomic coordinates and contain data reporting on the status of each cytosine base from a genomic sample or genomic regions of a genomic sample, including, but not limited to, data (a) for each cytosine base identifying the chromosome, genomic coordinate or position, strand, contextual sequence (e.g., CpG or other alternative contextual sequence), and trinucleotide context, and (b) number of cytosine bases that are methylated and number of cytosine bases that are not methylated. For instance, the cytosine report file may be a cytosine report from the MethylSeq software in a .CX or .TXT format. In the alternative to a cytosine report, in some embodiments, bias-adjusted-methylation-assay system 106 identifies counted numbers of nucleotide reads supporting methylated and unmethylated cytosine bases from other output data files, such as a FASTQ file or BCL file.

In some embodiments, the bias-adjusted-methylation-assay system 106 determines a first counted number of nucleotide reads supporting a methylated cytosine base and a second counted number of nucleotide reads supporting an unmethylated cytosine base for each target cytosine base. For instance, the bias-adjusted-methylation-assay system 106 identifies a first counted number of nucleotide reads supporting methylated cytosine sites and a second counted number of nucleotide reads supporting unmethylated cytosine sites based on (i) a cytosine report rile or (ii) a FASTQ file comprising data for an alignment or coverage between nucleotide reads for a genomic sample generated by the methylation sequencing assay 300 and particular cytosine bases at particular genomic coordinates in a reference genome.

As further shown in FIG. 3C, the bias-adjusted-methylation-assay system 106 predicts a first corrected number of nucleotide reads 316 supporting a methylated cytosine site and a second corrected number of nucleotide reads 318 supporting an unmethylated cytosine site-based on the false positive rate 308, the false negative rate 310, the true positive rate 314, the true negative rate 312, the first counted number of nucleotide reads, and the second counted number of nucleotide reads for a target cytosine base. For example, in some embodiments, the bias-adjusted-methylation-assay system 106 determines the first corrected number of nucleotide reads 316 supporting a methylated cytosine site by (i) determining a first difference between a first numerator product of the true negative rate 312 and the first counted number of nucleotide reads and a second numerator product of the false positive rate 308 and the second counted number of nucleotide reads, (ii) determining a second difference between a first denominator product of the true positive rate 314 and the true negative rate 312 and a second denominator product of the false negative rate 310 and the false positive rate 308, and (iii) determining a quotient of the first difference over the second difference.

To further illustrate, in some embodiments, the bias-adjusted-methylation-assay system 106 predicts the first corrected number of nucleotide reads 316 supporting a methylated cytosine site using the following function (1): $M = \frac{TNR ∗ m - FPR ∗ u}{TPR ∗ TNR - FNR ∗ FPR}$ (1) In function (1), *M* represents the first corrected number of nucleotide reads 316; *TNR, FPR, TPR,* and *FNR* represent the true negative rate 312, the false positive rate 308, the true positive rate 314, and the false negative rate 310, respectively; *m* represents a first counted number of nucleotide reads supporting methylated cytosine sites; and n represents a second counted number of nucleotide reads supporting unmethylated cytosine sites. As indicated above, the bias-adjusted-methylation-assay system 106 can predict a first corrected number of nucleotide reads supporting a methylated cytosine site for each target cytosine base using the relevant *m* and *n* for each cytosine position.

As a further example, in some embodiments, the bias-adjusted-methylation-assay system 106 determines the second corrected number of nucleotide reads 318 supporting an unmethylated cytosine site by (i) determining a first difference between a first numerator product of the true positive rate 314 and the second counted number of nucleotide reads and a second numerator product of the false negative rate 310 and the first counted number of nucleotide reads, (ii) determining a second difference between a first denominator product of the true positive rate 314 and the true negative rate 312 and a second denominator product of the true negative rate 312 and the false positive rate 308, and (iii) determining a quotient of the first difference over the second difference.

To further illustrate, in some embodiments, the bias-adjusted-methylation-assay system 106 predicts the second corrected number of nucleotide reads 318 supporting an unmethylated cytosine site using the following function (2): $U = \frac{TPR ∗ m - FNR ∗ u}{TPR ∗ TNR - TNR ∗ FPR}$ (2) In function (2), *U* represents the second corrected number of nucleotide reads 318; *TNR, FPR, TPR,* and *FNR* represent the true negative rate 312, the false positive rate 308, the true positive rate 314, and the false negative rate 310, respectively; *m* represents a first counted number of nucleotide reads supporting methylated cytosine sites; and *n* represents a second counted number of nucleotide reads supporting unmethylated cytosine sites. As indicated above, the bias-adjusted-methylation-assay system 106 can predict a second corrected number of nucleotide reads supporting an unmethylated cytosine site for each target cytosine base using the relevant *m* and *n* for each cytosine position.

Based on the first corrected number of nucleotide reads 316 and the second corrected number of nucleotide reads 318 for a target cytosine base, as further shown in FIG. 3C, the bias-adjusted-methylation-assay system 106 determines a corrected methylation-level value for the target cytosine base at a specific genomic coordinate within the sample nucleotide sequence 306a. For example, in some cases, the bias-adjusted-methylation-assay system 106 generates a corrected methylation-level value for a target cytosine base by determining a quotient of the first corrected number of nucleotide reads 316 for a target cytosine base at a particular genomic coordinate over a sum of the first corrected number of nucleotide reads 316 and the second corrected number of nucleotide reads 318 for the target cytosine base at the particular genomic coordinate.

To further illustrate, in some embodiments, the bias-adjusted-methylation-assay system 106 determines a corrected methylation-level value for a target cytosine base at a particular genomic coordinate using the following function (3): ${MLV}^{c} = \frac{M}{U + M}$ (3) In function (3), *MLV^{c}* represents a corrected methylation-level value for a target cytosine base at a particular genomic coordinate, *M* represents the first corrected number of nucleotide reads 316 for the target cytosine base at the particular genomic coordinate, and *U* represents the second corrected number of nucleotide reads 318 for the target cytosine base at the particular genomic coordinate.

As indicated above, in some embodiments, the bias-adjusted-methylation-assay system 106 determines corrected methylation-level values 322a, 322b, 322c through 322n for respective target cytosine bases at genomic coordinates indicated in a table 324 by using function (3). The bias-adjusted-methylation-assay system 106 also identifies methylation-level values 320a, 320b, 320c through 320n initially determined by the methylation sequencing assay 300 for the respective target cytosine bases at the genomic coordinates indicated in the table 324.

Although not depicted in FIG. 3C, in some embodiments, the bias-adjusted-methylation-assay system 106 determines a corrected methylation-level value specific to a contextual sequence flanking a target cytosine base. In some cases, for example, the bias-adjusted-methylation-assay system 106 uses artificial oligonucleotides comprising known contextual sequences flanking methylated or unmethylated cytosine bases. Such an artificial oligonucleotide may be, for instance, a methylated artificial oligonucleotide comprising one or more cytosine bases flanked by a contextual sequence and an unmethylated artificial oligonucleotide comprising one or more cytosine bases flanked by the same or different contextual sequence.

Consistent with the disclosure above, the bias-adjusted-methylation-assay system 106 runs the methylated artificial oligonucleotide and unmethylated artificial oligonucleotide through the methylation sequencing assay 300 to determine a number of methylated cytosine bases and a number of unmethylated cytosine bases converted into uracil or thymine bases. Based on the expected and actual conversions of cytosine bases from the methylated artificial oligonucleotide and the unmethylated artificial oligonucleotide, the bias-adjusted-methylation-assay system 106 determines a false positive rate, a false negative rate, a true positive rate, and a true negative rate specific to a contextual sequence. Based on such false and true rates and counted numbers of nucleotide reads supporting a methylated cytosine base and an unmethylated cytosine base corresponding to the target cytosine base, the bias-adjusted-methylation-assay system 106 determines a corrected methylation-level value specific to the contextual sequence flanking the target cytosine base by using functions (1), (2), and (3) above.

In the alternative to determining false and true rates using artificial oligonucleotides comprising contextual sequences, in some embodiments, the bias-adjusted-methylation-assay system 106 identifies historical false positive and false negative rates predetermined for a given methylation sequencing assay and a contextual sequence. In accordance with one or more embodiments, FIG. 3D illustrates the bias-adjusted-methylation-assay system accessing or identifying, from a database, predetermined false and true rates at which a methylation sequencing assay converts cytosine bases flanked by a contextual sequence and determining a corrected methylation-level value for a target cytosine base specific to the contextual sequence flanking the target cytosine base.

As shown in FIG. 3D, for instance, the bias-adjusted-methylation-assay system either identifies the methylation-level value 320a previously determined by the methylation sequencing assay 300 or performs the methylation sequencing assay 300 to determine the methylation-level value 320a. For instance, in some cases, the bias-adjusted-methylation-assay system 106 performs the methylation sequencing assay 300 for the sample nucleotide sequence 306a by (i) enzymatically converting methylated cytosine bases within the sample nucleotide sequence 306a into uracil bases or thymine bases; (ii) determining base calls of nucleotide reads 326 for the sample nucleotide sequence 306a and/or other sample nucleotide sequences from a genomic sample using the sequencing device 114; and (iii) comparing the base calls from the nucleotide reads 326 to a reference genome 328 or non-enzymatically converted nucleotide reads to identify cytosine bases in the nucleotide reads 326 that have been converted into uracil bases or thymine bases and, therefore, indicate methylated cytosine bases at corresponding genomic coordinates.

As further shown in FIG. 3D, the bias-adjusted-methylation-assay system 106 identifies counted numbers of nucleotide reads supporting methylated or unmethylated cytosine sites. Based on a cytosine report file or the alignment between the nucleotide reads 326 and the reference genome 328, for instance, the bias-adjusted-methylation-assay system 106 identifies a first counted number of nucleotide reads 330 supporting a methylated cytosine base at the genomic coordinate for a target cytosine base and a second counted number of nucleotide reads 332 supporting an unmethylated cytosine base at the genomic coordinate for the target cytosine base. In some cases, the bias-adjusted-methylation-assay system 106 identifies the first counted number of nucleotide reads 330 and the second counted number of nucleotide reads 332 from a cytosine report file or other output file related to a methylation sequence assay. For instance, in some embodiments, the bias-adjusted-methylation-assay system 106 identifies, from the cytosine report file, a number for *m* representing a first counted number of nucleotide reads supporting methylated cytosine sites and a number for *n* representing a second counted number of nucleotide reads supporting unmethylated cytosine sites

In addition to identifying counted numbers of nucleotide reads supporting methylated or unmethylated cytosine sites, as further shown in FIG. 3D, the bias-adjusted-methylation-assay system 106 identifies predetermined rates 338 at which the methylation sequencing assay 300 converts cytosine bases flanked by a contextual sequence 336. In particular, the bias-adjusted-methylation-assay system 106 accesses, from a database 334, a false positive rate, a false negative rate, a true positive rate, and a true negative rate at which the methylation sequencing assay 300 converts cytosine bases flanked by the contextual sequence 336-as previously determined by either the bias-adjusted-methylation-assay system 106 or another computing system. As indicated above, in some embodiments, the database 334 stores data representing the contextual sequence 336 (e.g., single-letter code) flanking a target cytosine base and includes data pointers or mappings linked to the relevant false positive rate, false negative rate, true positive rate, and true negative rate.

Having accessed or determined the first counted number of nucleotide reads 330, the second counted number of nucleotide reads 332, and the predetermined rates 338, the bias-adjusted-methylation-assay system 106 determines the corrected methylation-level value 322a. By executing functions (1) and (2), for example, the bias-adjusted-methylation-assay system 106 predicts a first corrected number of nucleotide reads 340 supporting a methylated cytosine site flanked by the contextual sequence and the second corrected number of nucleotide reads 342 supporting an unmethylated cytosine site flanked by the contextual sequence-based on the first counted number of nucleotide reads 330, the second counted number of nucleotide reads 332, and the predetermined rates 338 as inputs. By executing function (3), the bias-adjusted-methylation-assay system 106 further determines the corrected methylation-level value 322a specific to the target cytosine base flanked by the contextual sequence 336 based on the first corrected number of nucleotide reads 340 and the second corrected number of nucleotide reads 342.

As further indicated by FIG. 3D, the bias-adjusted-methylation-assay system 106 can determine corrected methylation-level values for different target cytosine bases flanked by different contextual sequences within a sample nucleotide sequence. For each target cytosine base, for instance, the bias-adjusted-methylation-assay system 106 can (i) determine a first counted number of nucleotide reads supporting a methylated cytosine base at a different genomic coordinate for a different target cytosine base and a second counted number of nucleotide reads supporting an unmethylated cytosine base at the different genomic coordinate for the different target cytosine base; (ii) access or identify, from the database 334, predetermined rates at which a methylation sequencing assay converts cytosine bases flanked by a different contextual sequence; and (iii) determine a corrected methylation-level value for the different target cytosine base specific to the contextual sequence flanking the different target cytosine base.

As indicated above, the bias-adjusted-methylation-assay system 106 predicts corrected numbers of nucleotide reads supporting methylated and unmethylated cytosine sites as part of an efficient computational model. In accordance with one or more embodiments, FIG. 3E illustrates a comparison of counted nucleotide reads supporting methylated and unmethylated cytosine sites and corrected numbers of nucleotide reads supporting the same methylated and unmethylated cytosine sites. In particular, the bias-adjusted-methylation-assay system 106 applies a computational model to predict the first corrected number of nucleotide reads 340 supporting methylated cytosine sites within a sample nucleotide sequence and a second corrected number of nucleotide reads 342 supporting unmethylated cytosine sites within a sample nucleotide sequence.

As shown in FIG. 3E, the bias-adjusted-methylation-assay system 106 identifies the first counted number of nucleotide reads 330 supporting one or more methylated cytosine bases from target cytosine site(s) 344 within a genome of a genomic sample and the second counted number of nucleotide reads 332 supporting one or more unmethylated cytosine bases from target cytosine site(s) 344. As indicated above, the bias-adjusted-methylation-assay system 106 can identify such counted numbers of nucleotide reads from a cytosine report file or other output file for the methylation sequencing assay 300. In this illustration, the first counted number of nucleotide reads 330 comprises five nucleotide reads that, when compared to a reference genome or non-enzymatically converted nucleotide reads, support identifying methylated cytosine bases at the target cytosine site(s) 344. Further, the second counted number of nucleotide reads 332 comprises four nucleotide reads that, when compared to a reference genome or non-enzymatically converted nucleotide reads, support identifying unmethylated cytosine bases at the target cytosine site(s) 344. To illustrate multiple embodiments, the target cytosine site(s) 344 may be a single or multiple target cytosine sites at particular genomic coordinates.

As part of determining a corrected methylation-level value, in some embodiments, the bias-adjusted-methylation-assay system 106 executes functions (1) and (2) to predict the first corrected number of nucleotide reads 340 supporting one or more methylated cytosine bases from target cytosine site(s) 344 within a genome of the genomic sample and a second corrected number of nucleotide reads 342 one or more unmethylated cytosine bases from target cytosine site(s) 344. In this illustration, the first corrected number of nucleotide reads 340 comprises three nucleotide reads that support identifying methylated cytosine bases at the target cytosine site(s) 344. Further, the second corrected number of nucleotide reads 342 comprises six nucleotide reads that support identifying unmethylated cytosine bases at the target cytosine site(s) 344.

As shown in FIG. 3E, therefore, the first corrected number of nucleotide reads 340 and the second corrected number of nucleotide reads 342 differ from the first counted number of nucleotide reads 330 and the second counted number of nucleotide reads 332, respectively. As noted above, however, corrected numbers of nucleotide reads predicted to support methylated and unmethylated cytosine sites do not necessarily differ from counted numbers of nucleotide reads identified to support methylated and unmethylated cytosine sites. Depending on the false positive or negative rates and true positive or negative rates of a given methylation sequencing assay and the target cytosine base, in some embodiments, the bias-adjusted-methylation-assay system 106 predicts corrected numbers of nucleotide reads supporting methylated and unmethylated cytosine sites that confirm (or are the same as) counted numbers of nucleotide reads identified to support methylated and unmethylated cytosine sites.

As noted above, in some embodiments, the bias-adjusted-methylation-assay system 106 uses corrected methylation-level values to recover biological signals for certain disorders or diseases that would otherwise be missed by existing methylation sequencing assays. For instance, in some cases, the bias-adjusted-methylation-assay system 106 recovers biological signals for cancer, Alzheimer's, and other methylation-dependent diseases based on corrections to methylation-difference values for a differentially methylated region (DMR). In accordance with one or more embodiments, FIGS. 4A and 4B illustrate the bias-adjusted-methylation-assay system 106 modifying methylation-difference values for DMRs corresponding to target cytosine bases within a sample nucleotide sequence based on corrected methylation-level values. By modifying the methylation-difference values based on corrected methylation-level values, the bias-adjusted-methylation-assay system 106 generates modified methylation-difference values that are closer in value to ground-truth methylation-difference values than initial and uncorrected methylation-difference values.

To determine ground-truth methylation-level values (e.g., ground-truth beta values), researchers performed a bisulfite methylation sequencing assay on sample nucleotide sequences that correspond to a promoter genomic region for the B-cell CLL/lymphoma 9 (BCL9) gene on chromosome 1 and that were extracted from a normal genomic sample and a target genomic sample. The researchers likewise performed a given methylation sequencing assay using APOBEC enzyme on sample nucleotide sequences, from the normal genomic sample and the target genomic sample, that correspond to the BCL9 promoter region. Consistent with the disclosure above, the researchers also used the bias-adjusted-methylation-assay system 106 to determine corrected methylation-level values for target cytosine bases in the BCL9 promoter region based on data from the given methylation sequencing assay and the computational model described above (e.g., as depicted in FIG. 3C).

Based on the methylation-level values generated by the bisulfite methylation sequencing assay, the researchers determined ground-truth mean methylation-difference values between the normal genomic sample and the target genomic sample at DMR 406 and DMR 408. The researchers likewise determined (i) mean methylation-difference values between the normal genomic sample and the target genomic sample at DMR 406 and DMR 408 based on methylation-level values from the given methylation sequencing assay and (ii) corrected mean methylation-difference values between the normal genomic sample and the target genomic sample at DMR 406 and DMR 408 based on corrected methylation-level values from the bias-adjusted-methylation-assay system 106.

As shown in FIG. 4A, a graph 400a depicts (i) ground-truth mean methylation-difference values and mean methylation-difference values for the target genomic sample and (ii) mean methylation-difference values for the normal genomic sample along a mean methylation difference axis 402a and a base position axis 404a. As indicated by FIG. 4A, the graph 400a shows the mean methylation-difference values for the normal genomic sample at approximately 0.0 across base positions of the BCL9 promoter region, including at DMR 406 and DMR 408. By contrast, the graph 400a shows the mean methylation-difference values for the target genomic sample at approximately -0.55 at DMR 406 and at approximately 0.0 at DMR 408. The graph 400a further shows the ground-truth mean methylation-difference values for the target genomic sample at approximately -0.75 at DMR 406 and at approximately -0.65 at DMR 408. Accordingly, the mean methylation-difference values for the target genomic sample from the given methylation sequencing assay differ significantly from the ground-truth methylation-difference values at DMR 406 and DMR 408.

As shown in FIG. 4B, by contrast, a graph 400b depicts (i) ground-truth mean methylation-difference values and corrected mean methylation-difference values for the target genomic sample and (ii) mean methylation-difference values for the normal genomic sample along a mean methylation difference axis 402b and a base position axis 404b. As indicated by FIG. 4B, the graph 400b shows the mean methylation-difference values for the normal genomic sample at approximately 0.0 across base positions of the BCL9 promoter region, including at DMR 406 and DMR 408. By contrast, the graph 400b shows the corrected mean methylation-difference values for the target genomic sample at approximately -0.85 at DMR 406 and at approximately -0.65 at DMR 408. Just like the graph 400a in FIG. 4A, the graph 400b in FIG. 4B further shows the ground-truth mean methylation-difference values for the target genomic sample at approximately -0.75 at DMR 406 and at approximately -0.65 at DMR 408.

As shown by a comparison of the graphs 400a and 400b in FIGS. 4A and 4B, the corrected mean methylation-difference values for the target genomic sample generated by the bias-adjusted-methylation-assay system 106 come much closer to the ground-truth methylation-difference values at DMR 406 and DMR 408 than the mean methylation-difference values for the target genomic sample from the given methylation sequencing assay. Indeed, methylation-difference values at DMR 406 and DMR 408 within the BCL9 promoter region represent biological signals for breast cancer. By modifying mean methylation-difference values based on corrected methylation-level values, therefore, the bias-adjusted-methylation-assay system 106 recovers biological signals for cancer that would have otherwise been missed by biological signals based on uncorrected methylation-level values from the given methylation sequencing assay.

As further indicated above, after determining corrected methylation-level values, the bias-adjusted-methylation-assay system 106 can provide, for display within a graphical user interface, a visualization of initial or uncorrected methylation-level values and corrected methylation-level values. In accordance with one or more embodiments, FIG. 5 depicts the bias-adjusted-methylation-assay system 106 generating data for graphics representing (i) initial or uncorrected methylation-level values determined by a methylation-sequencing assay and (ii) corrected methylation-level values determined by the bias-adjusted-methylation-assay system 106. In particular, a computing device 500 presents, within a graphical user interface 501, a graph 502 showing ground-truth methylation-level values (e.g., ground-truth beta values), methylation-level values initially determined by a methylation sequencing assay, and corrected methylation-level values at genomic coordinates for a promoter genomic region. While the bias-adjusted-methylation-assay system 106 comprises instructions that (upon execution) cause the computing device 500 to present the graph 502 shown in FIG. 5, this disclosure will either refer to the computing device 500 of the bias-adjusted-methylation-assay system 106 as performing certain actions described below for simplicity without repeatedly describing such computer-executable instructions.

To determine and compare uncorrected and corrected methylation-level values to ground-truth methylation-level values, researchers performed Enzymatic Methyl-seq (EM-seq) as a methylation sequencing assay on sample nucleotide sequences that (i) correspond to a promoter genomic region for the B-cell CLL/lymphoma 9 (BCL9) gene on chromosome 1 of a human and that (ii) were extracted from a genomic sample. For instance, the researchers performed EM-seq as described by Romualdas Vaisvila et al., Enzymatic Methyl Sequencing Detects DNA Methylation at Single-Base Resolution from Picograms of DNA, 30 Genome Research 1280-1289 (2021). The methylation-level values (e.g., cytosine report file beta values) from EM-seq were treated as ground-truth methylation-level values. The researchers likewise performed a given methylation sequencing assay using an APOBEC enzyme on sample nucleotide sequences that correspond to the BCL9 promoter region. Consistent with the disclosure above, the researchers also used the bias-adjusted-methylation-assay system 106 to determine corrected methylation-level values based on data from the given methylation sequencing assay and the computational model described above (e.g., as depicted in FIG. 3C).

As shown in FIG. 5, the computing device 500 presents the graph 502 comprising a methylation-level-value axis 504 and a base position axis 506. As shown along the methylation-level-value axis 504, the graph 502 includes the ground-truth methylation-level values, uncorrected or initial methylation-level values determined by the given methylation sequencing assay, and the corrected methylation-level values determined by the bias-adjusted-methylation-assay system 106. Along the base position axis 506, the graph 502 depicts genomic coordinates or base positions for target cytosine bases (within the BCL9 promoter region) at which methylation-level values were determined.

As indicated by the graph 502, from genomic coordinates or base positions 2,100 to 4,300, the corrected methylation-level values exhibit approximately as good or better accuracy than the methylation-level values in comparison to the ground-truth methylation-level values. By contrast, from genomic coordinates or base positions 1,000 to 2,000, the corrected methylation-level values and the methylation-level values exhibit mixed relative accuracy in comparison to the ground-truth methylation-level values. Together, the graph 502 depicts a visualization of both methylation-level values and corrected methylation-level values at genomic coordinates for particular target cytosine bases.

As further indicated above, the bias-adjusted-methylation-assay system 106 improves the accuracy of detecting methylation levels of cytosine bases within a sample nucleotide sequence. In accordance with one or more embodiments, FIGS. 6A and 6B depict histograms comparing either uncorrected methylation-level values determined by a given methylation sequencing assay or corrected methylation-level values determined by the bias-adjusted-methylation-assay system 106 to ground-truth methylation-level values across genomic regions of a chromosome. As illustrated by a comparison of the graphs in FIGS. 6A and 6B, the corrected methylation-level values exhibit a distribution that better matches the ground-truth methylation-level values than the uncorrected methylation-level values.

To determine and compare uncorrected and corrected methylation-level values to ground-truth methylation-level values, researchers performed EM-seq as a methylation sequencing assay on sample nucleotide sequences, from a genomic sample, that correspond to genomic regions across chromosome 1 of a human. The methylation-level values from EM-seq were treated as ground-truth methylation-level values. The researchers likewise performed a given methylation sequencing assay using an APOBEC enzyme on sample nucleotide sequences, from the genomic sample, that correspond to genomic regions across chromosome 1. Consistent with the disclosure above, the researchers also used the bias-adjusted-methylation-assay system 106 to determine corrected methylation-level values based on data from the given methylation sequencing assay and the computational model described above (e.g., as depicted in FIG. 3C).

As shown in FIG. 6A, a histogram 600a depicts ground-truth methylation-level values determined by EM-seq as a methylation sequencing assay and uncorrected methylation-level values determined by the given methylation-sequencing assay across a CpG density axis 602a and a methylation-level-values axis 604a. Along the CpG density axis 602a, the histogram 600a shows CpG density values for a number of CpG sites that belong or contribute to a given methylation-level value (e.g., a given beta value) across chromosome 1. In particular, for the histogram 600a, the CpG density values represent frequency of CpG site at a given beta value over a product of a total number of CpG sites and bin width. Along the methylation-level-values axis 604a, the histogram 600a shows beta values between 0.0 and 1.0. The histogram 600a represents an overlap of distribution between uncorrected methylation-level values and ground-truth methylation-level values with a diagonal pattern. As indicated by diagonal pattern in the histogram 600a, the distribution of uncorrected methylation-level values across CpG density does not match the distribution of ground-truth methylation-level values. For example, between beta values 0.8 and 1.0, the histogram 600a shows little overlap between the distribution of uncorrected methylation-level values and the distribution of ground-truth methylation-level values.

As shown in FIG. 6B, by contrast, a histogram 600b depicts ground-truth methylation-level values determined by EM-seq as a methylation sequencing assay and corrected methylation-level values determined by the bias-adjusted-methylation-assay system 106 across a CpG density axis 602b and a methylation-level-values axis 604b. Along the CpG density axis 602b, the histogram 600b shows CpG density values for a number of CpG sites that belong or contribute to a given methylation-level value (e.g., a given beta value) across chromosome 1. In particular, for the histogram 600b, the CpG density values represent frequency of CpG site at a given beta value over a product of a total number of CpG sites and bin width. Along the methylation-level-values axis 604b, the histogram 600b shows beta values between 0.0 and 1.0. The histogram 600b represents an overlap of distribution between corrected methylation-level values and ground-truth methylation-level values with a diagonal pattern. As indicated by diagonal pattern in the histogram 600b, the distribution of corrected methylation-level values across CpG density matches the distribution of ground-truth methylation-level values better than the distribution of uncorrected methylation-level values in the histogram 600a. For example, between beta values 0.0 and 1.0, the histogram 600b shows significant overlap between the distribution of corrected methylation-level values and the distribution of ground-truth methylation-level values-including at the most problematic section for uncorrected methylation-level values between values 0.8 and 1.0.

Turning now to FIG. 7, this figure illustrates a flowchart of a series of acts 700 of utilizing a computational model to determine a corrected methylation-level value for a target cytosine base within a sample nucleotide sequence in accordance with one or more embodiments of the present disclosure. While FIG. 7 illustrates acts according to one embodiment, alternative embodiments may omit, add to, reorder, and/or modify any of the acts shown in FIG. 7. The acts of FIG. 7 can be performed as part of a method. Alternatively, a non-transitory computer readable storage medium can comprise instructions that, when executed by one or more processors, cause a computing device or a system to perform the acts depicted in FIG. 7. In still further embodiments, a system comprising at least one processor and a non-transitory computer readable medium comprising instructions that, when executed by one or more processors, cause the system to perform the acts of FIG. 7.

As shown in FIG. 7, the acts 700 include an act 702 of identifying, for a methylation sequencing assay, a methylation-level value for a target cytosine base within a sample nucleotide sequence. In particular, in some embodiments, the act 702 includes identifying, for a methylation sequencing assay, a methylation-level value indicating a level of methylation of a target cytosine base within a sample nucleotide sequence. In some cases, the sample nucleotide sequence is extracted from a non-human organism.

As further shown in FIG. 7, the acts 700 include an act 704 of determining a false positive rate and a false negative rate at which the methylation sequencing assay converts cytosine bases. In particular, in some embodiments, the act 704 includes determining a false positive rate and a false negative rate at which the methylation sequencing assay converts cytosine bases within nucleotide sequences. For example, in certain embodiments, determining the false positive rate and the false negative rate comprises determining the false positive rate and the false negative rate at which the methylation sequencing assay converts cytosine bases into uracil bases or thymine bases.

As suggested above, in some embodiments, determining the false positive rate or the false negative rate comprises estimating the false positive rate or the false negative rate at which the methylation sequencing assay converts cytosine bases flanked by a contextual sequence. In some such embodiments, generating the corrected methylation-level value comprises generating the corrected methylation-level value for the target cytosine base specific to the contextual sequence flanking the target cytosine base.

Relatedly, in certain cases, determining the false positive rate comprises estimating a rate at which the methylation sequencing assay incorrectly converts one or more unmethylated cytosine bases within a given nucleotide sequence into one or more uracil bases or thymine bases; and determining the false negative rate comprises estimating a rate at which the methylation sequencing assay fails to convert one or more methylated cytosine bases within a given nucleotide sequence into one or more uracil bases or thymine bases.

To further illustrate, in some implementations, determining the false positive rate at which the methylation sequencing assay converts cytosine bases comprises: converting, utilizing the methylation sequencing assay, unmethylated cytosine bases within an unmethylated artificial oligonucleotide; and comparing a number of converted unmethylated cytosine bases to a total number of the unmethylated cytosine bases within the unmethylated artificial oligonucleotide. Conversely, in some embodiments, determining the false negative rate at which the methylation sequencing assay converts cytosine bases comprises: converting, utilizing the methylation sequencing assay, methylated cytosine bases within a methylated artificial oligonucleotide; and comparing a number of converted methylated cytosine bases to a total number of the methylated cytosine bases within the methylated artificial oligonucleotide.

As further shown in FIG. 7, the acts 700 include an act 706 of predicting a first corrected number of nucleotide reads supporting methylated cytosine sites and a second corrected number of nucleotide reads supporting unmethylated cytosine sites. In particular, in certain implementations, the act 706 includes, based on the false positive rate and the false negative rate, predicting a first corrected number of nucleotide reads supporting methylated cytosine sites within the sample nucleotide sequence and a second corrected number of nucleotide reads supporting unmethylated cytosine sites within the sample nucleotide sequence.

As suggested above, in some embodiments, predicting the first corrected number of nucleotide reads or the second corrected number of nucleotide reads comprises: determining a true positive rate and a true negative rate at which the methylation sequencing assay converts cytosine bases within nucleotide sequences; identifying, from data generated by the methylation sequencing assay, a first counted number of nucleotide reads supporting methylated cytosine sites within the sample nucleotide sequence and a second counted number of nucleotide reads supporting unmethylated cytosine sites within the sample nucleotide sequence; and predicting the first corrected number of nucleotide reads or the second corrected number of nucleotide reads based on the false positive rate, the false negative rate, the true positive rate, the true negative rate, the first counted number of nucleotide reads, and the second counted number of nucleotide reads.

Relatedly, in one or more embodiments, predicting the first corrected number of nucleotide reads supporting the methylated cytosine sites within the sample nucleotide sequence comprises: determining a first difference between a first numerator product of the true negative rate and the first counted number of nucleotide reads and a second numerator product of the false positive rate and the second counted number of nucleotide reads; determining a second difference between a first denominator product of the true positive rate and the true negative rate and a second denominator product of the false negative rate and the false positive rate; and determining a quotient of the first difference over the second difference.

Conversely, in one or more embodiments, predicting the second corrected number of nucleotide reads supporting the unmethylated cytosine sites within the sample nucleotide sequence comprises: determining a first difference between a first numerator product of the true positive rate and the second counted number of nucleotide reads and a second numerator product of the false negative rate and the first counted number of nucleotide reads; determining a second difference between a first denominator product of the true positive rate and the true negative rate and a second denominator product of the true negative rate and the false positive rate; and determining a quotient of the first difference over the second difference.

Further, in certain implementations, predicting the first corrected number of nucleotide reads comprises determining a number of nucleotide reads supporting methylated cytosine sites within at least a first nucleotide sequence of the nucleotide sequences; and predicting the second corrected number of nucleotide reads comprises determining a number of nucleotide reads supporting unmethylated cytosine sites within at least a second nucleotide sequence of the nucleotide sequences.

As further shown in FIG. 7, the acts 700 include an act 708 of generating a corrected methylation-level value for the target cytosine base within the sample nucleotide sequence. In particular, in certain embodiments, the act 708 includes generating a corrected methylation-level value that corrects for a bias reflected in the methylation-level value for the target cytosine base within the sample nucleotide sequence based on the first corrected number of nucleotide reads and the second corrected number of nucleotide reads. For example, in certain embodiments, generating the corrected methylation-level value comprises determining a quotient of the first corrected number of nucleotide reads over a sum of the first corrected number of nucleotide reads and the second corrected number of nucleotide reads.

In addition or in the alternative to the acts 702-708, in certain implementations, the acts 700 include determining that a counted number of nucleotide reads covering the target cytosine base within the sample nucleotide sequence fails to satisfy a coverage threshold; and based on the counted number of nucleotide reads failing to satisfy the coverage threshold, generating the corrected methylation-level value for the target cytosine base.

As suggested above, in addition or in the alternative, in some embodiments, the acts 700 include changing, based on the corrected methylation-level value, a methylation-difference value for a differentially methylated region corresponding to the target cytosine base within the sample nucleotide sequence. Further, in some cases, the acts 700 include providing, for display within a graphical user interface, the methylation-level value and the corrected methylation-level value.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleobase type from another are particularly applicable. In some embodiments, the process to determine the nucleotide sequence of a target nucleic acid (i.e., a nucleic-acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using γ-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320). In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026. This approach is being commercialized by Solexa (now Illumina Inc.), and is also described in WO 91/06678 and WO 07/123,744. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005). Other approaches have separated the terminator chemistry from the cleavage of the fluorescence label (Ruparel et al., Proc Natl Acad Sci USA 102: 5932-7 (2005). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372.

Some embodiments can utilize detection of four different nucleotides using fewer than four different labels. For example, SBS can be performed utilizing methods and systems described in U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597.

Some embodiments can utilize nanopore sequencing (Deamer, D. W. & Akeson, M. "Nanopores and nucleic acids: prospects for ultrarapid sequencing." Trends Biotechnol. 18, 147151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003). In such embodiments, the target nucleic acid passes through a nanopore. The nanopore can be a synthetic pore or biological membrane protein, such as a-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and y-phosphate-labeled nucleotides as described, for example, in U.S. Pat. No. 7,329,492 and U.S. Pat. No. 7,211,414 or nucleotide incorporations can be detected with zero-mode waveguides as described, for example, in U.S. Pat. No. 7,315,019 and using fluorescent nucleotide analogs and engineered polymerases as described, for example, in U.S. Pat. No. 7,405,281 and U.S. Patent Application Publication No. 2008/0108082. The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 Al; US 2009/0127589 Al; US 2010/0137143 Al; or US 2010/0282617 Al. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 Al and US Ser. No. 13/273,666. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666.

The sequencing system described above sequences nucleic-acid polymers present in samples received by a sequencing device. As defined herein, "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free circulating DNA. In some embodiments, the sample can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the bias-adjusted-methylation-assay system 106 can include software, hardware, or both. For example, the components of the bias-adjusted-methylation-assay system 106 can include one or more instructions stored on a computer-readable storage medium and executable by processors of one or more computing devices (e.g., the user client device 110). When executed by the one or more processors, the computer-executable instructions of the bias-adjusted-methylation-assay system 106 can cause the computing devices to perform the bubble detection methods described herein. Alternatively, the components of the bias-adjusted-methylation-assay system 106 can comprise hardware, such as special purpose processing devices to perform a certain function or group of functions. Additionally, or alternatively, the components of the bias-adjusted-methylation-assay system 106 can include a combination of computer-executable instructions and hardware.

Furthermore, the components of the bias-adjusted-methylation-assay system 106 performing the functions described herein with respect to the bias-adjusted-methylation-assay system 106 may, for example, be implemented as part of a stand-alone application, as a module of an application, as a plug-in for applications, as a library function or functions that may be called by other applications, and/or as a cloud-computing model. Thus, components of the bias-adjusted-methylation-assay system 106 may be implemented as part of a stand-alone application on a personal computing device or a mobile device. Additionally, or alternatively, the components of the bias-adjusted-methylation-assay system 106 may be implemented in any application that provides sequencing services including, but not limited to Illumina BaseSpace, BeadArray, BeadChip, Illumina DRAGEN, Infinium Methylation Assay, or Illumina TruSight software. "Illumina," "BeadArray," "BeadChip," "BaseSpace," "DRAGEN," "Infinium Methylation Assay," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phase-change memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general-purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 8 illustrates a block diagram of a computing device 800 that may be configured to perform one or more of the processes described above. One will appreciate that one or more computing devices such as the computing device 800 may implement the bias-adjusted-methylation-assay system 106 and the sequencing system 104. As shown by FIG. 8, the computing device 800 can comprise a processor 802, a memory 804, a storage device 806, an I/O interface 808, and a communication interface 810, which may be communicatively coupled by way of a communication infrastructure 812. In certain embodiments, the computing device 800 can include fewer or more components than those shown in FIG. 8. The following paragraphs describe components of the computing device 800 shown in FIG. 8 in additional detail.

In one or more embodiments, the processor 802 includes hardware for executing instructions, such as those making up a computer program. As an example, and not by way of limitation, to execute instructions for dynamically modifying workflows, the processor 802 may retrieve (or fetch) the instructions from an internal register, an internal cache, the memory 804, or the storage device 806 and decode and execute them. The memory 804 may be a volatile or nonvolatile memory used for storing data, metadata, and programs for execution by the processor(s). The storage device 806 includes storage, such as a hard disk, flash disk drive, or other digital storage device, for storing data or instructions for performing the methods described herein.

The I/O interface 808 allows a user to provide input to, receive output from, and otherwise transfer data to and receive data from computing device 800. The I/O interface 808 may include a mouse, a keypad or a keyboard, a touch screen, a camera, an optical scanner, network interface, modem, other known I/O devices or a combination of such I/O interfaces. The I/O interface 808 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 808 is configured to provide graphical data to a display for presentation to a user. The graphical data may be representative of one or more graphical user interfaces and/or any other graphical content as may serve a particular implementation.

The communication interface 810 can include hardware, software, or both. In any event, the communication interface 810 can provide one or more interfaces for communication (such as, for example, packet-based communication) between the computing device 800 and one or more other computing devices or networks. As an example, and not by way of limitation, the communication interface 810 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 810 may facilitate communications with various types of wired or wireless networks. The communication interface 810 may also facilitate communications using various communication protocols. The communication infrastructure 812 may also include hardware, software, or both that couples components of the computing device 800 to each other. For example, the communication interface 810 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms without departing from its essential characteristics. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description. All changes that come within the meaning and range of equivalency of the claims are to be embraced within their scope.

## Claims

1. A computer-implemented method comprising:
identifying, for a methylation sequencing assay, a methylation-level value indicating a level of methylation of a target cytosine base within a sample nucleotide sequence;
determining a false positive rate at which the methylation sequencing assay converts cytosine bases within nucleotide sequences by comparing (i) a number of converted unmethylated cytosine bases within an unmethylated artificial oligonucleotide subject to the methylation sequencing assay to (ii) a total number of unmethylated cytosine bases within the unmethylated artificial oligonucleotide;
determining a false negative rate at which the methylation sequencing assay converts cytosine bases within nucleotide sequences by comparing (i) a number of unconverted methylated cytosine bases within a methylated artificial oligonucleotide subject to the methylation sequencing assay to (ii) a total number of methylated cytosine bases within the methylated artificial oligonucleotide;
predicting, based on a first quotient comparing the false positive rate to the false negative rate, a first corrected number of nucleotide reads supporting methylated cytosine sites within the sample nucleotide sequence;
predicting, based on a second quotient comparing the false negative rate to the false positive rate, a second corrected number of nucleotide reads supporting unmethylated cytosine sites within the sample nucleotide sequence; and
generating a corrected methylation-level value that corrects for a bias reflected in the methylation-level value for the target cytosine base within the sample nucleotide sequence by determining a third quotient comparing (i) the first corrected number of nucleotide reads to (ii) the first corrected number of nucleotide reads and the second corrected number of nucleotide reads.

2. The computer-implemented method of claim 1, wherein:
determining the false positive rate or the false negative rate comprises estimating the false positive rate or the false negative rate at which the methylation sequencing assay converts cytosine bases flanked by a contextual sequence; and
generating the corrected methylation-level value for the target cytosine base specific to the contextual sequence flanking the target cytosine base.

3. The computer-implemented method of claim 1 or 2, wherein:
determining the false positive rate comprises estimating a rate at which the methylation sequencing assay incorrectly converts one or more unmethylated cytosine bases within a given nucleotide sequence into one or more uracil bases or thymine bases; and
determining the false negative rate comprises estimating a rate at which the methylation sequencing assay fails to convert one or more methylated cytosine bases within a given nucleotide sequence into one or more uracil bases or thymine bases.

4. The computer-implemented method of any one of claims 1-3, wherein determining the false positive rate at which the methylation sequencing assay converts cytosine bases comprises:
converting, utilizing the methylation sequencing assay, one or more of the unmethylated cytosine bases within the unmethylated artificial oligonucleotide.

5. The computer-implemented method of any one of claims 1-4, wherein determining the false negative rate at which the methylation sequencing assay converts cytosine bases comprises:
converting, utilizing the methylation sequencing assay, one or more of the methylated cytosine bases within the methylated artificial oligonucleotide.

6. The computer-implemented method of any one of claims 1-5, wherein predicting the first corrected number of nucleotide reads or the second corrected number of nucleotide reads comprises:
determining a true positive rate and a true negative rate at which the methylation sequencing assay converts cytosine bases within nucleotide sequences by comparing actual conversions and expected conversions for the cytosine bases;
identifying, from data generated by the methylation sequencing assay, a first counted number of nucleotide reads supporting methylated cytosine sites within the sample nucleotide sequence and a second counted number of nucleotide reads supporting unmethylated cytosine sites within the sample nucleotide sequence; and
predicting the first corrected number of nucleotide reads based on determining the first quotient by further comparing the false positive rate to the false negative rate, the true positive rate, the true negative rate, the first counted number of nucleotide reads, and the second counted number of nucleotide reads; or
predicting the second corrected number of nucleotide reads based on determining the second quotient by further comparing, the false negative rate to the false positive rate, the true positive rate, the true negative rate, the first counted number of nucleotide reads, and the second counted number of nucleotide reads.

7. The computer-implemented method of claim 6, wherein predicting the first corrected number of nucleotide reads supporting the methylated cytosine sites within the sample nucleotide sequence comprises:
determining a first difference between a first numerator product of the true negative rate and the first counted number of nucleotide reads and a second numerator product of the false positive rate and the second counted number of nucleotide reads;
determining a second difference between a first denominator product of the true positive rate and the true negative rate and a second denominator product of the false negative rate and the false positive rate; and
determining the first quotient by determining a quotient of the first difference over the second difference.

8. The computer-implemented method of claim 6, wherein predicting the second corrected number of nucleotide reads supporting the unmethylated cytosine sites within the sample nucleotide sequence comprises:
determining a first difference between a first numerator product of the true positive rate and the second counted number of nucleotide reads and a second numerator product of the false negative rate and the first counted number of nucleotide reads;
determining a second difference between a first denominator product of the true positive rate and the true negative rate and a second denominator product of the true negative rate and the false positive rate; and
determining the second quotient by determining a quotient of the first difference over the second difference.

9. The computer-implemented method of any one of claims 1-8, wherein:
predicting the first corrected number of nucleotide reads comprises determining a number of nucleotide reads supporting methylated cytosine sites within at least a first nucleotide sequence of the nucleotide sequences; and
predicting the second corrected number of nucleotide reads comprises determining a number of nucleotide reads supporting unmethylated cytosine sites within at least a second nucleotide sequence of the nucleotide sequences.

10. The computer-implemented method of any one of claims 1-9, wherein generating the corrected methylation-level value comprises determining the third quotient by determining a quotient of the first corrected number of nucleotide reads over a sum of the first corrected number of nucleotide reads and the second corrected number of nucleotide reads.

11. The computer-implemented method of any one of claims 1-10, further comprising:
determining that a counted number of nucleotide reads covering the target cytosine base within the sample nucleotide sequence fails to satisfy a coverage threshold; and
based on the counted number of nucleotide reads failing to satisfy the coverage threshold, generating the corrected methylation-level value for the target cytosine base.

12. The computer-implemented method of any one of claims 1-11, further comprising changing, based on the corrected methylation-level value, a methylation-difference value for a differentially methylated region corresponding to the target cytosine base within the sample nucleotide sequence.

13. The computer-implemented method of any one of claims 1-12, further comprising, providing, for display within a graphical user interface, the methylation-level value and the corrected methylation-level value.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a computing device to perform a method according to any one of claims 1-13.

## Patentansprüche

1. Computerimplementiertes Verfahren, das Folgendes umfasst:
Identifizieren, für einen Methylierungsequenzierungstest, eines Methylierungsgradwerts, der einen Methylierungsgrad einer Ziel-Cytosinbase innerhalb einer Proben-Nukleotidsequenz angibt;
Bestimmen einer Falsch-Positiv-Rate, mit der der Methylierungssequenzierungstest Cytosinbasen innerhalb von Nukleotidsequenzen umwandelt, durch Vergleichen (i) einer Anzahl umgewandelter unmethylierter Cytosinbasen innerhalb eines unmethylierten künstlichen Oligonukleotids, das dem Methylierungssequenzierungstest unterzogen wird, mit (ii) einer Gesamtanzahl unmethylierter Cytosinbasen innerhalb des unmethylierten künstlichen Oligonukleotids;
Bestimmen einer Falsch-Negativ-Rate, mit der der Methylierungssequenzierungstest Cytosinbasen innerhalb von Nukleotidsequenzen umwandelt, durch Vergleichen (i) einer Anzahl nicht umgewandelter methylierter Cytosinbasen in einem methylierten künstlichen Oligonukleotid, das dem Methylierungssequenzierungstest unterzogen wird, mit (ii) einer Gesamtanzahl methylierter Cytosinbasen in dem methylierten künstlichen Oligonukleotid;
Vorhersagen, basierend auf einem ersten Quotienten, der die Falsch-Positiv-Rate mit der Falsch-Negativ-Rate vergleicht, einer ersten korrigierten Anzahl von Nukleotid-Reads, die methylierte Cytosinstellen innerhalb der Proben-Nukleotidsequenz unterstützen;
Vorhersagen, basierend auf einem zweiten Quotienten, der die Falsch-Negativ-Rate mit der Falsch-Positiv-Rate vergleicht, einer zweiten korrigierten Anzahl von Nukleotid-Reads, die unmethylierte Cytosinstellen innerhalb der Proben-Nukleotidsequenz unterstützen; und
Erzeugen eines korrigierten Methylierungsgradwerts, der eine Verzerrung in dem Methylierungsgradwert der Ziel-Cytosinbase innerhalb der Proben-Nukleotidsequenz korrigiert, indem ein dritter Quotient bestimmt wird, der (i) die erste korrigierte Anzahl von Nukleotid-Reads mit (ii) der ersten korrigierten Anzahl von Nukleotid-Reads und der zweiten korrigierten Anzahl von Nukleotid-Reads vergleicht.

2. Computerimplementiertes Verfahren nach Anspruch 1, wobei:
das Bestimmen der Falsch-Positiv-Rate oder der Falsch-Negativ-Rate das Schätzen der Falsch-Positiv-Rate oder der Falsch-Negativ-Rate umfasst, mit der der Methylierungssequenzierungstest Cytosinbasen umwandelt, die von einer Kontextsequenz flankiert sind; und
Erzeugen des korrigierten Methylierungsgradwerts für die Ziel-Cytosinbase, die für die Kontextsequenz, die die Ziel-Cytosinbase flankiert, spezifisch ist.

3. Computerimplementiertes Verfahren nach Anspruch 1 oder 2, wobei:
das Bestimmen der Falsch-Positiv-Rate das Schätzen einer Rate, mit der der Methylierungssequenzierungstest fälschlicherweise eine oder mehrere unmethylierte Cytosinbasen innerhalb einer gegebenen Nukleotidsequenz in eine oder mehrere Uracilbasen oder Thyminbasen umwandelt, umfasst; und
das Bestimmen der Falsch-Negativ-Rate das Schätzen einer Rate umfasst, mit der der Methylierungssequenzierungstest nicht in der Lage ist, eine oder mehrere methylierte Cytosinbasen innerhalb einer gegebenen Nukleotidsequenz in eine oder mehrere Uracilbasen oder Thyminbasen umzuwandeln.

4. Computerimplementiertes Verfahren nach einem der Ansprüche 1-3, wobei das Bestimmen der Falsch-Positiv-Rate, mit der der Methylierungssequenzierungstest Cytosinbasen umwandelt, Folgendes umfasst:
Umwandeln, unter Einsatz des Methylierungssequenzierungstests, einer oder mehrerer unmethylierter Cytosinbasen innerhalb des unmethylierten künstlichen Oligonukleotids.

5. Computerimplementiertes Verfahren nach einem der Ansprüche 1-4, wobei das Bestimmen der Falsch-Negativ-Rate, mit der der Methylierungssequenzierungstest Cytosinbasen umwandelt, Folgendes umfasst:
Umwandeln, unter Einsatz des Methylierungssequenzierungstests, einer oder mehrerer methylierter Cytosinbasen innerhalb des methylierten künstlichen Oligonukleotids.

6. Computerimplementiertes Verfahren nach einem der Ansprüche 1-5, wobei das Vorhersagen der ersten korrigierten Anzahl von Nukleotid-Reads oder der zweiten korrigierten Anzahl von Nukleotid-Reads Folgendes umfasst:
Bestimmen einer Richtig-Positiv-Rate und einer Richtig-Negativ-Rate, mit der der Methylierungssequenzierungstest Cytosinbasen innerhalb von Nukleotidsequenzen umwandelt, durch Vergleichen tatsächlicher Umwandlungen mit erwarteten Umwandlungen für die Cytosinbasen;
Identifizieren, aus Daten, die durch den Methylierungsequenzierungstest erzeugt werden, einer ersten gezählten Anzahl von Nukleotid-Reads, die methylierte Cytosinstellen innerhalb der Proben-Nukleotidsequenz unterstützen, und einer zweiten gezählten Anzahl von Nukleotid-Reads, die unmethylierte Cytosinstellen innerhalb der Proben-Nukleotidsequenz unterstützen; und
Vorhersagen der ersten korrigierten Anzahl von Nukleotid-Reads basierend auf dem Bestimmen des ersten Quotienten durch weiteres Vergleichen der Falsch-Positiv-Rate mit der Falsch-Negativ-Rate, der Richtig-Positiv-Rate, der Richtig-Negativ-Rate, der ersten gezählten Anzahl von Nukleotid-Reads und der zweiten gezählten Anzahl von Nukleotid-Reads; oder
Vorhersagen der zweiten korrigierten Anzahl von Nukleotid-Reads basierend auf dem Bestimmen des zweiten Quotienten durch weiteres Vergleichen der Falsch-Negativ-Rate mit der Falsch-Positiv-Rate, der Richtig-Positiv-Rate, der Richtig-Negativ-Rate, der ersten gezählten Anzahl von Nukleotid-Reads und der zweiten gezählten Anzahl von Nukleotid-Reads.

7. Computerimplementiertes Verfahren nach Anspruch 6, wobei das Vorhersagen der ersten korrigierten Anzahl von Nukleotid-Reads, die die methylierten Cytosin-Stellen innerhalb der Proben-Nukleotidsequenz unterstützen, Folgendes umfasst:
Bestimmen einer ersten Differenz zwischen einem ersten Produkt im Zähler der Richtig-Negativ-Rate und der ersten gezählten Anzahl von Nukleotid-Reads und einem zweiten Produkt im Zähler der Falsch-Positive-Rate und der zweiten gezählten Anzahl von Nukleotid-Reads;
Bestimmen einer zweiten Differenz zwischen einem ersten Produkt im Nenner der Richtig-Positiv-Rate und der Richtig-Negativ-Rate und einem zweiten Produkt im Nenner der Falsch-Negativ-Rate und der Falsch-Positiv-Rate; und
Bestimmen des ersten Quotienten durch Bestimmen eines Quotienten der ersten Differenz über der zweiten Differenz.

8. Computerimplementiertes Verfahren nach Anspruch 6, wobei das Vorhersagen der zweiten korrigierten Anzahl von Nukleotid-Reads, die die unmethylierten Cytosin-Stellen innerhalb der Proben-Nukleotidsequenz unterstützen, Folgendes umfasst:
Bestimmen einer ersten Differenz zwischen einem ersten Produkt im Zähler der Richtig-Positive-Rate und der zweiten gezählten Anzahl von Nukleotid-Reads und einem zweiten Produkt im Zähler der Falsch-Negative-Rate und der ersten gezählten Anzahl von Nukleotid-Reads;
Bestimmen einer zweiten Differenz zwischen einem ersten Produkt im Nenner der Richtig-Positiv-Rate und der Richtig-Negativ-Rate und einem zweiten Produkt im Nenner der Richtig-Negativ-Rate und der Falsch-Positiv-Rate; und
Bestimmen des zweiten Quotienten durch Bestimmen eines Quotienten der ersten Differenz über der zweiten Differenz.

9. Computerimplementiertes Verfahren nach einem der Ansprüche 1-8, wobei:
das Vorhersagen der ersten korrigierten Anzahl von Nukleotid-Reads das Bestimmen einer Anzahl von Nukleotid-Reads umfasst, die methylierte Cytosin-Stellen innerhalb mindestens einer ersten Nukleotidsequenz der Nukleotidsequenzen unterstützen; und
das Vorhersagen der zweiten korrigierten Anzahl von Nukleotid-Reads das Bestimmen einer Anzahl von Nukleotid-Reads umfasst, die unmethylierte Cytosin-Stellen innerhalb mindestens einer zweiten Nukleotidsequenz der Nukleotidsequenzen unterstützen.

10. Computerimplementiertes Verfahren nach einem der Ansprüche 1-9, wobei das Erzeugen des korrigierten Methylierungsgradwerts das Bestimmen des dritten Quotienten durch Bestimmen eines Quotienten der ersten korrigierten Anzahl von Nukleotid-Reads über einer Summe der ersten korrigierten Anzahl von Nukleotid-Reads und der zweiten korrigierten Anzahl von Nukleotid-Reads umfasst.

11. Computerimplementiertes Verfahren nach einem der Ansprüche 1-10, das weiter Folgendes umfasst:
Bestimmen, dass eine gezählte Anzahl von Nukleotid-Reads, die die Ziel-Cytosinbase innerhalb der Probennukleotidsequenz abdecken, einen Abdeckungsschwellenwert nicht erfüllt; und
basierend auf der gezählten Anzahl von Nukleotid-Reads, die den Abdeckungsschwellenwert nicht erfüllen, Erzeugen des korrigierten Methylierungsgradwerts für die Ziel-Cytosinbase.

12. Computerimplementiertes Verfahren nach einem der Ansprüche 1-11, das weiter das Ändern, basierend auf dem korrigierten Methylierungsgradwert, eines Methylierungsdifferenzwerts für einen differenziell methylierten Bereich, der der Ziel-Cytosinbase innerhalb der Proben-Nukleotidsequenz entspricht, umfasst.

13. Computerimplementiertes Verfahren nach einem der Ansprüche 1-12, das weiter das Bereitstellen des Methylierungsgradwerts und des korrigierten Methylierungsgradwerts zum Anzeigen in einer grafischen Benutzeroberfläche umfasst.

14. System, das mindestens einen Prozessor und ein nichtflüchtiges, computerlesbares Medium umfasst, das Anweisungen umfasst, die, wenn sie von dem mindestens einen Prozessor ausgeführt werden, das System veranlassen, ein Verfahren nach einem der Ansprüche 1-13 durchzuführen.

15. Nichtflüchtiges, computerlesbares Medium, das Anweisungen speichert, die, wenn sie von mindestens einem Prozessor ausgeführt werden, eine Rechenvorrichtung veranlassen, ein Verfahren nach einem der Ansprüche 1-13 durchzuführen.

## Revendications

1. Procédé mis en œuvre par ordinateur comprenant :
l'identification, pour une analyse de séquençage de la méthylation, d'une valeur de niveau de méthylation indiquant un niveau de méthylation d'une base de cytosine cible dans une séquence nucléotidique d'un échantillon ;
la détermination d'un taux de faux positifs auquel l'analyse de séquençage de la méthylation convertit des bases de cytosine dans des séquences nucléotidiques en comparant (i) un nombre de bases de cytosine non méthylée converties dans un oligonucléotide artificiel non méthylé soumis à l'analyse de séquençage de la méthylation à (ii) un nombre total de bases de cytosine non méthylée dans l'oligonucléotide artificiel non méthylé ;
la détermination d'un taux de faux négatifs auquel l'analyse de séquençage de la méthylation convertit des bases de cytosine dans des séquences nucléotidiques en comparant (i) un nombre de bases de cytosine méthylée non converties dans un oligonucléotide artificiel méthylé soumis à l'analyse de séquençage de la méthylation à (ii) un nombre total de bases de cytosine méthylée dans l'oligonucléotide artificiel méthylé ;
la prédiction, sur la base d'un premier quotient comparant le taux de faux positifs au taux de faux négatifs, d'un premier nombre corrigé de lectures des nucléotides confirmant la présence de sites de cytosine méthylée dans la séquence nucléotidique de l'échantillon ;
la prédiction, sur la base d'un deuxième quotient comparant le taux de faux négatifs au taux de faux positifs, d'un second nombre corrigé de lectures des nucléotides confirmant la présence de sites de cytosine non méthylée dans la séquence nucléotidique de l'échantillon ; et
la génération d'une valeur corrigée du niveau de méthylation qui corrige un biais reflété dans la valeur du niveau de méthylation pour la base de cytosine cible dans la séquence nucléotidique de l'échantillon en déterminant un troisième quotient comparant (i) le premier nombre corrigé de lectures des nucléotides au (ii) premier nombre corrigé de lectures des nucléotides et au deuxième nombre corrigé de lectures des nucléotides.

2. Procédé mis en œuvre par ordinateur selon la revendication 1, dans lequel :
la détermination du taux de faux positifs ou du taux de faux négatifs comprend l'estimation du taux de faux positifs ou du taux de faux négatifs auquel l'analyse de séquençage de la méthylation convertit des bases de cytosine entourées d'une séquence contextuelle ;
la génération de la valeur corrigée du niveau de méthylation pour la base de cytosine cible spécifique à la séquence contextuelle entourant la base cytosine cible.

3. Procédé mis en œuvre par ordinateur selon la revendication 1 ou 2, dans lequel :
la détermination du taux de faux positifs comprend l'estimation d'un taux auquel l'analyse de séquençage de la méthylation convertit incorrectement une ou plusieurs bases de cytosine non méthylée au sein d'une séquence nucléotidique donnée en une ou plusieurs bases d'uracile ou de thymine ;
la détermination du taux de faux négatifs comprend l'estimation d'un taux auquel l'analyse de séquençage de la méthylation ne parvient pas à convertir une ou plusieurs bases de cytosine méthylée au sein d'une séquence nucléotidique donnée en une ou plusieurs bases d'uracile ou de thymine.

4. Procédé mis en œuvre par ordinateur selon l'une quelconque des revendications 1-3, dans lequel la détermination du taux de faux positifs auquel l'analyse de séquençage de la méthylation convertit des bases de cytosine comprend :
la conversion, au moyen de l'analyse de séquençage de la méthylation, d'une ou plusieurs des bases de cytosine non méthylée dans l'oligonucléotide artificiel non méthylé.

5. Procédé mis en œuvre par ordinateur selon l'une quelconque des revendications 1-4, dans lequel la détermination du taux de faux négatifs auquel l'analyse de séquençage de la méthylation convertit des bases de cytosine comprend :
la conversion, au moyen de l'analyse de séquençage de la méthylation, d'une ou plusieurs des bases de cytosine méthylée dans l'oligonucléotide artificiel méthylé.

6. Procédé mise en œuvre par ordinateur selon l'une quelconque des revendications 1-5, dans lequel la prédiction du premier nombre corrigé de lectures de nucléotides ou du deuxième nombre corrigé de lectures de nucléotides comprend :
la détermination d'un taux de vrais positifs et d'un taux de vrais négatifs auxquels l'analyse de séquençage de la méthylation convertit des bases de cytosine dans des séquences nucléotidiques en comparant des conversions réelles et des conversions attendues pour les bases de cytosine ;
l'identification, à partir de données générées par l'analyse de séquençage de la méthylation, d'un premier nombre compté de lectures de nucléotides confirmant la présence de sites de cytosine méthylée dans la séquence nucléotidique de l'échantillon et d'un second nombre compté de lectures de nucléotides confirmant la présence de sites de cytosine non méthylé dans la séquence nucléotidique de l'échantillon ;
la prédiction du premier nombre corrigé de lectures de nucléotides sur la base de la détermination du premier quotient en comparant plus finement le taux de faux positifs au taux de faux négatifs, au taux de vrais positifs, au taux de vrais négatifs, au premier nombre compté de lectures de nucléotides comptées et au second nombre compté de lectures de nucléotides ; ou
la prédiction du second nombre corrigé de lectures de nucléotides sur la base de la détermination du deuxième quotient en comparant plus finement le taux de faux négatifs au taux de faux positifs, au taux de vrais positifs, au taux de vrais négatifs, au premier nombre compté de lectures de nucléotides et au second nombre compté de lectures de nucléotides.

7. Procédé mis en œuvre par ordinateur selon la revendication 6, dans lequel la prédiction du premier nombre corrigé de lectures de nucléotides confirmant la présence de sites de cytosine méthylé dans la séquence nucléotidique de l'échantillon comprend :
la détermination d'une première différence entre un premier produit des numérateurs du taux de vrais négatifs et du premier nombre compté de lectures de nucléotides et un second produit des numérateurs du taux de faux positifs et du second nombre compté de lectures de nucléotides ;
la détermination d'une seconde différence entre un premier produit des dénominateurs du taux de vrais positifs et du taux de vrais négatifs et un second produit des dénominateurs du taux de faux négatifs et du taux de faux positifs ; et
la détermination du premier quotient en déterminant un quotient de la première différence sur la seconde différence.

8. Procédé mis en œuvre par ordinateur selon la revendication 6, dans lequel la prédiction du second nombre corrigé de lectures de nucléotides confirmant la présence de sites de cytosine non méthylé dans la séquence nucléotidique de l'échantillon comprend :
la détermination d'une première différence entre un premier produit des numérateurs du taux de vrais positifs et du second nombre compté de lectures de nucléotides et un second produit des numérateurs du taux de faux négatifs et du premier nombre compté de lectures de nucléotides ;
la détermination d'une seconde différence entre un premier produit des dénominateurs du taux de vrais positifs et du taux de vrais négatifs et un second produit des dénominateurs du taux de vrais négatifs et du taux de faux positifs ; et
la détermination du deuxième quotient en déterminant un quotient de la première différence sur la seconde différence.

9. Procédé mis en œuvre par ordinateur selon l'une quelconque des revendications 1-8, dans lequel :
la prédiction du premier nombre corrigé de lectures de nucléotides comprend la détermination d'un nombre de lectures de nucléotides confirmant la présence de sites de cytosine méthylée dans au moins une première séquence nucléotidique des séquences nucléotidiques ; et
la prédiction du second nombre corrigé de lectures de nucléotides comprend la détermination d'un nombre de lectures de nucléotides confirmant la présence de sites de cytosine non méthylé dans au moins une deuxième séquence nucléotidique des séquences nucléotidiques.

10. Procédé mis en œuvre par ordinateur selon l'une quelconque des revendications 1-9, dans lequel la génération de la valeur corrigée du niveau de méthylation comprend la détermination du troisième quotient en déterminant un quotient du premier nombre corrigé de lectures de nucléotides sur une somme du premier nombre corrigé de lectures de nucléotides et du second nombre corrigé de lectures de nucléotides.

11. Procédé mis en œuvre par ordinateur selon l'une quelconque des revendications 1-10, comprenant en outre :
la détermination qu'un nombre compté de lectures de nucléotides couvrant la base de cytosine cible dans la séquence nucléotidique de l'échantillon ne satisfait pas à un seuil de couverture ; et
sur la base du fait que le nombre compté de lectures de nucléotides ne satisfait pas le seuil de couverture, la génération de la valeur corrigée du niveau de méthylation pour la base de cytosine cible.

12. Procédé mis en œuvre par ordinateur selon l'une quelconque des revendications 1-11, comprenant en outre la modification, sur la base de la valeur corrigée du niveau de méthylation, d'une valeur de différence de méthylation pour une région différentiellement méthylée correspondant à la base de cytosine cible dans la séquence nucléotidique de l'échantillon.

13. Procédé mis en œuvre par ordinateur selon l'une quelconque des revendications 1-12, comprenant en outre la fourniture, pour affichage dans une interface utilisateur graphique, de la valeur du niveau de méthylation et de la valeur corrigée du niveau de méthylation.

14. Système comprenant au moins un processeur et un support non transitoire lisible par ordinateur comprenant des instructions qui, lorsqu'elles sont exécutées par le au moins un processeur, amènent le système à réaliser un procédé selon l'une quelconque des revendications 1-13.

15. Support non transitoire lisible par ordinateur stockant des instructions qui, lorsqu'elles sont exécutées par au moins un processeur, amènent un dispositif informatique à réaliser un procédé selon l'une quelconque des revendications 1-13.
